# EUROPEAN PATENT SPECIFICATION

(11) **EP 3 930 628 B1**
(45) Date of publication and mention of the grant of the patent: **02.08.2023**
(21) Application number: 20709898.9
(22) Date of filing: 06.02.2020
(51) Int. Cl.: A61F 2/24

(54) **DOUBLE HEART VALVE ANCHORING**
DOPPELTE HERZKLAPPENVERANKERUNG
ANCRAGE DE VALVULE CARDIAQUE DOUBLE

(30) Priority: 27.02.2019 US 201962811453 P
(43) Date of publication of application: 05.01.2022
(73) Proprietor: Edwards Lifesciences Corporation, Irvine, CA 92614 (US)
(72) Inventor: NIA, Nima, V., Irvine, CA 92614 (US); CAO, Hengchu, Irvine, CA 92614 (US); TRIPATHY, Sakyasingh, Irvine, CA 92614 (US); DOMINICK, Douglas, Thomas, Irvine, CA 92614 (US); SU, Bingquan, Irvine, CA 92614 (US)
(74) Representative: Eisenführ Speiser
(86) International application number: PCT/US2020/016928
(87) International publication number: WO 2020/176208

(56) References cited:
- EP-A1- 3 184 081
- WO-A1-2016/108181
- US-A1- 2013 261 738

## Description

### BACKGROUND

### Field

The present disclosure generally relates to the field of medical implant devices.

### Description of Related Art

Implantation of heart valve devices can be performed to address various heart valve abnormalities. Implantation of both a tricuspid valve device and a mitral valve device can be advantageous for patients suffering from dysfunction in both valves.

EP 3 184081 A1 discloses a prosthetic mitral valve coaptation enhancement device comprising a main body consisting of a stent frame and a valve element attached thereto, wherein the main body has a sealing section and a valve-bearing section, the valve-bearing section carrying the valve element. The shape of the sealing section of the main body, in the compressed state, has a form that is adapted to the coaptation zone of the native mitral valve during systole, and the radial rigidity of the sealing section of the stent frame is lower than the radial rigidity of the valve-bearing section. The prosthetic mitral valve coaptation enhancement device further comprises at least one anchoring element, which anchoring element is coupled to the main body and is capable to anchor the main body within the native mitral valve region of a heart.

### SUMMARY

Described herein are one or more methods not belonging to the claimed invention and/or devices to anchor both a tricuspid valve device and a mitral valve device to the septum. In some implementations, the present disclosure relates to a double heart valve anchoring system comprising a first anchor which comprises at least a portion configured to be secured within a first trans-septal opening in an interventricular septum, a tricuspid valve device coupled to the first anchor, and a mitral valve device coupled to the first anchor.

In some embodiments, the tricuspid valve device comprises a tricuspid valve replacement and the mitral valve device comprises a mitral valve replacement. The first connector can extend between the tricuspid valve replacement and the mitral valve replacement, coupling the tricuspid valve replacement and the mitral valve replacement to the first anchor, and being configured to extend through the first trans-septal opening.

In some embodiments, the double heart valve anchoring system can comprise a second connector extending between the tricuspid valve replacement and the mitral valve replacement, coupling the tricuspid valve replacement and the mitral valve replacement. Both the first connector and the second connector can couple the tricuspid valve replacement and the mitral valve replacement to the first anchor, and both the first connector and the second connector can be configured to extend through the first trans-septal opening.

In some embodiments, the double heart valve anchoring system can comprise a second anchor comprising at least a portion configured to be secured within a second trans-septal opening in the interventricular septum, and wherein the second connector couples the tricuspid valve replacement and the mitral valve replacement to the second anchor, and wherein the second connector is configured to extend through the second trans-septal opening.

Respective ends of at least one of the first connector and the second connector can be coupled to a portion of the tricuspid valve replacement configured to be positioned within a right ventricle and to a portion of the mitral valve replacement configured to be positioned within a left ventricle. In some embodiments, respective ends of at least one of the first connector and the second connector is coupled to a distal end of the tricuspid valve replacement configured to be positioned within the right ventricle and to a distal end of the mitral valve replacement configured to be positioned within the left ventricle.

In some embodiments, a length of at least one of the first connector and the second connector is adjustable. The double heart valve anchoring system can comprise at least one locking mechanism to lock at least one of the first connector and the second connector at a respective selected length.

In some embodiments, a length of at least one of the first connector and the second connector is selected prior to implantation.

In some embodiments, at least one of the first connector and the second connector comprises a nitinol wire. In some embodiments, at least one of the first connector and the second connector is a flexible cord.

In some embodiments, at least one of the first connector and the second connector comprises a rigid connector portion, the rigid connector portion being configured to extend through the septum and into at least one of a right ventricle and a left ventricle to provide predetermined angles of septum anchoring for the tricuspid valve replacement and the mitral valve replacement.

In some embodiments, at least one of the first anchor and the second anchor comprises a rigid anchor portion, the rigid anchor portion being configured to extend into at least one of a right ventricle and a left ventricle to provide predetermined angles of septum anchoring for the tricuspid valve replacement and the mitral valve replacement.

In some embodiments, the tricuspid valve device comprises a tricuspid valve replacement and the mitral valve device comprises a mitral valve repair.

In some embodiments, the tricuspid valve device comprises a tricuspid valve repair and the mitral valve device comprises a mitral valve replacement.

In some embodiments, the tricuspid valve device comprises a tricuspid valve repair and the mitral valve device comprises a mitral valve repair. The tricuspid valve repair can comprise a first connector configured to couple a tricuspid valve leaflet to the first anchor, and the mitral valve repair can comprise a second connector coupling a mitral valve leaflet to the first anchor. In some embodiments, the double heart valve anchoring system comprises a connector coupling the tricuspid valve repair to the mitral valve repair, the connector being configured to couple to the first anchor and to extend through the first trans-septal opening. In some embodiments, the tricuspid valve repair and the mitral valve repair comprise the connector, the connector being configured to couple a tricuspid valve leaflet and a mitral valve leaflet to the first anchor, and to extend through the first trans-septal opening.

In some implementations, the present disclosure relates to a double heart valve anchoring system comprising a tricuspid valve replacement, a mitral valve replacement, and a first connector coupling the tricuspid valve replacement and the mitral valve replacement, and being configured to extend through a first trans-septal opening in an interventricular septum.

In some embodiments, the double heart valve anchoring system can comprise a second connector coupling the tricuspid valve replacement and the mitral valve replacement. In some embodiments, both the first connector and the second connector are configured to extend through the first trans-septal opening. In some embodiments, the second connector is configured to extend through a second trans-septal opening.

Respective ends of at least one of the first connector and the second connector can be coupled to a portion of the tricuspid valve replacement configured to be positioned within a right ventricle and to a portion of the mitral valve replacement configured to be positioned within a left ventricle. Respective ends of at least one of the first connector and the second connector can be coupled to a distal end of the tricuspid valve replacement configured to be positioned within the right ventricle and to a distal end of the mitral valve replacement configured to be positioned within the left ventricle.

In some embodiments, a length of at least one of the first connector and the second connector is adjustable. The double heart valve anchoring system can comprise at least one locking mechanism to lock at least one of the first connector and the second connector at a respective selected length.

In some embodiments, a length of at least one of the first connector and the second connector is selected prior to implantation.

In some embodiments, at least one of the first connector and the second connector comprises a nitinol wire. In some embodiments, at least one of the first connector and the second connector is a flexible cord.

In some embodiments, at least one of the first connector and the second connector comprises a rigid connector portion, the rigid connector portion being configured to extend through the interventricular septum and into at least one of the right ventricle and the left ventricle to provide predetermined angles of septum anchoring for the tricuspid valve replacement and the mitral valve replacement.

In some implementations, the present disclosure relates to a method of replacing a tricuspid valve and a mitral valve. The method can comprise introducing a delivery catheter carrying a double heart valve anchoring system through an inferior vena cava or a superior vena cava, and into a right atrium. The double heart valve anchoring system can comprise a tricuspid valve replacement, a mitral valve replacement and a first connector coupling the tricuspid valve replacement and the mitral valve replacement. The delivery catheter can be advanced from the right atrium into the right ventricle via an opening formed by a native tricuspid valve. The delivery catheter can then be threaded through a trans-septal opening in an interventricular septum to insert the delivery catheter into a left ventricle from the right ventricle. A distal end of the delivery catheter can be positioned at a target site for the mitral valve replacement, and the mitral valve replacement can be released at the target site for the mitral valve replacement. The delivery catheter can be retracted through the left ventricle, the trans-septal opening and into the right ventricle. Respective portions of the first connector can be released in the left ventricle, the trans-septal opening and the right ventricle while retracting the delivery catheter through the left ventricle, the trans-septal opening and into the right ventricle. A distal end of the delivery catheter can be positioned at a target site for the tricuspid valve replacement after retracting the delivery catheter into the right ventricle, and the tricuspid valve replacement can be released at the target site for the tricuspid valve replacement.

In some embodiments, introducing the delivery catheter comprises introducing the delivery catheter through the inferior vena cava.

In some embodiments, introducing the delivery catheter comprises introducing a double heart valve anchoring system comprising an anchor, the anchor being between the tricuspid valve replacement and the mitral valve replacement and coupled to the first connector. Retracting the delivery catheter can comprise releasing at least a portion of the anchor in the trans-septal opening. Releasing at least a portion of the anchor in the trans-septal opening can comprise sealing the trans-septal opening.

In some embodiments, introducing the delivery catheter comprises introducing a delivery catheter carrying a double heart valve anchoring system comprising a second connector coupling the tricuspid valve replacement and the mitral valve replacement. The method can comprise releasing respective portions of the second connector in the left ventricle, the trans-septal opening and the right ventricle while retracting the delivery catheter through the left ventricle, the trans-septal opening and into the right ventricle.

In some embodiments, the method can comprise adjusting a length of at least one of the first connector and the second connector after introducing the delivery catheter. A locking mechanism can be activated to secure at least one of the first connector and the second connector at a respective selected length after adjusting the length.

For purposes of summarizing the disclosure, certain aspects, advantages and novel features have been described herein. It is to be understood that not necessarily all such advantages may be achieved in accordance with any particular embodiment. Thus, the disclosed embodiments may be carried out in a manner that achieves or optimizes one advantage or group of advantages as taught herein without necessarily achieving other advantages as may be taught or suggested herein.

### BRIEF DESCRIPTION OF THE DRAWINGS

Throughout the drawings, reference numbers may be reused to indicate correspondence between reference elements. However, it should be understood that the use of similar reference numbers in connection with multiple drawings does not necessarily imply similarity between respective embodiments associated therewith. Furthermore, it should be understood that the features of the respective drawings are not necessarily drawn to scale, and the illustrated sizes thereof are presented for the purpose of illustration of inventive aspects thereof. Generally, certain of the illustrated features may be relatively smaller than as illustrated in some embodiments or configurations.
Figure 1 is a cross-sectional view of a human heart.
Figure 2 is a schematic diagram of an example of a double heart valve anchoring system comprising two heart valve replacements.
Figure 3 is a schematic diagram showing the double heart valve anchoring system of Figure 2 positioned at its target site in the heart.
Figure 4 is a schematic diagram of an example of a double heart valve anchoring system which does not include an anchor, the double heart valve anchoring system being positioned at its target site.
Figure 5A is a schematic diagram showing an example of a double heart valve anchoring system comprising an adjustable connector, the double heart valve anchoring system being positioned at its target site.
Figure 5B is a schematic diagram of an example of a locking mechanism for securing an adjustable connector at a selected length.
Figure 6 is a schematic diagram of a double heart valve anchoring system which includes a connector comprising a rigid or semi-rigid portion, the double heart valve anchoring system being positioned at its target site.
Figure 7A is a schematic diagram of a double heart valve anchoring system which includes an anchor comprising respective rigid or semi-rigid portions configured to extend into the left and right ventricles, the double heart valve anchoring system being positioned at its target site.
Figure 7B is a schematic diagram of the anchor described with reference to Figure 7A.
Figure 8 is a schematic diagram of a double heart valve anchoring system comprising three connectors, the double heart valve anchoring system being positioned at its target site.
Figure 9 is a schematic diagram of a double heart valve anchoring system comprising three connectors and three anchors, the double heart valve anchoring system being positioned at its target site.
Figure 10 is a schematic diagram of an example of a double heart valve anchoring system comprising a tricuspid valve repair and a mitral valve repair, the double heart valve anchoring system being positioned at its target site.
Figure 11 is a schematic diagram of an example of a double heart valve anchoring system comprising a tricuspid valve replacement and a mitral valve repair, the double heart valve anchoring system being positioned at its target site.
Figure 12 is a schematic diagram of a double heart valve anchoring system comprising an example of a tricuspid valve replacement and an example of a mitral valve replacement, the double heart valve anchoring system being positioned at its target site.
Figures 13A and 13B are schematic diagrams showing an example of a path along which a delivery catheter carrying a double heart valve anchoring system can be advanced and retracted.
Figures 14A and 14B are schematic diagrams showing another example of a path along which a delivery catheter carrying a double heart valve anchoring system can be advanced and retracted.
Figure 15 is a flow diagram of an example of an implantation procedure.

### DETAILED DESCRIPTION

The headings provided herein are for convenience only and do not necessarily affect the scope or meaning of the claimed invention.

The present disclosure relates to systems, devices, and methods for anchoring both a tricuspid valve device and a mitral valve device to the septum.

Although certain preferred embodiments and examples are disclosed below, inventive subject matter extends beyond the specifically disclosed embodiments to other alternative embodiments and/or uses and to modifications and equivalents thereof. Thus, the scope of the claims that may arise herefrom is not limited by any of the particular embodiments described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

Certain standard anatomical terms of location are used herein to refer to the anatomy of animals, and namely humans, with respect to the preferred embodiments. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

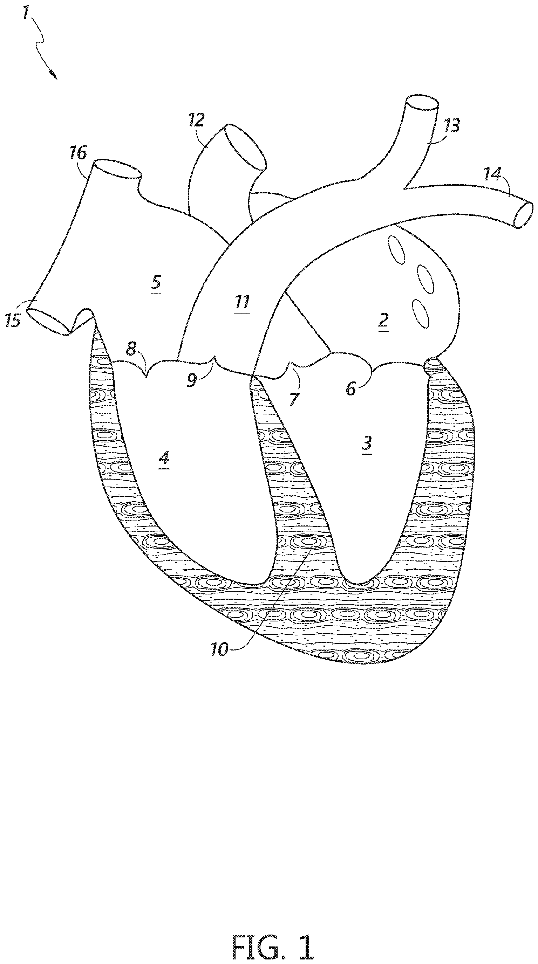

Various features of a heart 1 are described with reference to Figure 1 to assist in understanding the present disclosure. The heart 1 includes four chambers, namely the left atrium 2, the left ventricle 3, the right ventricle 4, and the right atrium 5. A wall of muscle, referred to as the septum 10, separates the left atrium 2 and right atrium 5, and the left ventricle 3 and right ventricle 4. Blood flow through the heart 1 is at least partially controlled by four valves, the mitral valve 6, aortic valve 7, tricuspid valve 8, and pulmonary valve 9. The mitral valve 6 separates the left atrium 2 and the left ventricle 3 and controls blood flow therebetween. The aortic valve 7 separates and controls blood flow between the left ventricle 3 and the aorta 12. The tricuspid valve 8 separates the right atrium 5 and the right ventricle 4 and controls blood flow therebetween. The pulmonary valve 9 separates the right ventricle 4 and the pulmonary artery 11, controlling blood flow therebetween.

The heart valves may generally comprise a relatively dense fibrous ring, referred to herein as the annulus, as well as a plurality of cusps or leaflets attached to the annulus. For example, the tricuspid valve 8, aortic valve 7 and pulmonary valve 9 generally have three cusps or leaflets. The mitral valve 6 generally has two leaflets or cusps. The size of the leaflets or cusps may be such that the leaflets or cusps coapt to close the valves, and move apart to open the valves. The atrioventricular (i.e., mitral and tricuspid) heart valves may further comprise a collection of chordae tendineae and papillary muscles (not shown) for securing the leaflets of the respective valves to promote and/or facilitate proper coaptation of the valve leaflets and prevent prolapse thereof. The papillary muscles, for example, may generally comprise finger-like projections from the ventricle wall. The valve leaflets are connected to the papillary muscles by the chordae tendineae.

In a healthy heart, the heart valves can properly open and close in response to a pressure gradient present during various stages of the cardiac cycle (e.g., relaxation and contraction) to at least partially control the flow of blood to a respective region of the heart and/or to blood vessels. Deoxygenated blood arriving from the rest of the body generally flows into the right side of the heart for transport to the lungs, and oxygenated blood from the lungs generally flows into the left side of the heart for transport to the rest of the body.

During ventricular diastole (e.g., when the ventricular heart muscles are relaxed), a negative pressure differential between the right ventricle 4 and the right atrium 5 can push leaflets of the tricuspid valve 8 apart to open the tricuspid valve 8, allowing deoxygenated blood in the right atrium 5 arriving from the inferior vena cava 15 and superior vena cava 16 to flow into the right ventricle 4. Leaflets of the pulmonary valve 9 are coapted during ventricular diastole, remaining in apposition to each other to keep the pulmonary valve 9 closed. A negative pressure differential between the left ventricle 3 and the left atrium 2 can push leaflets of the mitral valve 6 apart during ventricular diastole to open the mitral valve 6, allowing oxygenated blood in the left atrium 2 arriving from the pulmonary veins to flow into the left ventricle 3. Leaflets of the aortic valve 7 can remain coapted during ventricular diastole, keeping the aortic valve 7 closed.

During ventricular systole (e.g., when the ventricular heart muscles contract), a positive pressure differential between the right ventricle 4 and the right atrium 5 can keep leaflets of the tricuspid valve 8 coapted, or in apposition with one another, to close the tricuspid valve 8. Properly coapted leaflets can prevent leakage of blood from the right ventricle 4 into the right atrium 5. Meanwhile, leaflets of the pulmonary valve 9 can be pushed apart during ventricular systole, sending deoxygenated blood from the right ventricle 4 into the pulmonary artery 11 for transport to the lungs. The pulmonary artery 11 can carry deoxygenated blood from the right side of the heart to the lungs. The pulmonary artery 11 can include a pulmonary trunk and left 14 and right 13 pulmonary arteries that branch off of the pulmonary trunk, as shown. A positive pressure differential between the left ventricle 3 and the left atrium 2 during ventricular systole can keep leaflets of the mitral valve 6 coapted, or in apposition with one another, to close the mitral valve 6. Properly coapted mitral valve leaflets can prevent leakage of blood from the left ventricle 3 into the left atrium 2. Leaflets of the aortic valve 7 can be pushed apart during ventricular systole to allow flow of oxygenated blood from the left ventricle 3 to the aorta 12 for transport to the rest of the body.

Diseased heart valve and/or associated leaflets (e.g., tricuspid valve and/or mitral valve dysfunction) can result in valve leakage and/or other health complications. Valve stenosis can cause a valve to become narrowed or obstructed. Tricuspid valve stenosis and mitral valve stenosis can restrict blood flow from the right atrium to the right ventricle, and from the left atrium to the left ventricle, respectively. Valve regurgitation occurs when a valve does not close properly. For example, regurgitation can occur due to improper coaptation of the valve leaflets. Tricuspid valve regurgitation can result in blood flow leakage back into the right atrium from the right ventricle when the right ventricle contracts. Leakage of blood back into the right atrium can result in ineffective pumping of deoxygenated blood into the lungs. Mitral valve regurgitation can result in blood flow leakage back into the left atrium from the left ventricle when the left ventricle contracts. If regurgitation is severe enough, the heart may enlarge to maintain forward flow of blood, causing heart failure, such as when the heart does not pump enough blood to the body. This may produce symptoms ranging from shortness of breath during exertion, coughing, congestion around the heart and lungs, swelling of the legs and feet. Valve repair can be performed to address various valve diseases. In more severe cases, valve replacement can be performed.

The present disclosure relates to devices, systems for anchoring both a tricuspid valve device and a mitral valve device to one another on the interventricular septum. The tricuspid valve device comprises a tricuspid valve replacement or a tricuspid valve repair. The mitral valve device comprises a mitral valve replacement or a mitral valve repair. In a deployed state, a double heart valve anchoring system described herein comprises a mitral valve repair or replacement, and a tricuspid valve repair or replacement, tethered to one another through the septum. The double heart valve anchoring system comprises a connector configured to extend through a trans-septal opening, tethering a tricuspid valve replacement and a mitral valve replacement to one another and coupling the tricuspid valve replacement and mitral valve replacement to the septum. The double heart valve anchoring system comprises an anchor comprising at least a portion configured to be positioned within the trans-septal opening. The connector is coupled to the anchor, for example comprising a portion extending through an opening in the anchor.

In some embodiments, a mitral valve repair and a tricuspid valve repair can be coupled to one another via the trans-septal opening, anchoring the mitral valve repair and tricuspid valve repair to the septum and to one another. In some embodiments, a mitral valve repair and a tricuspid valve replacement can be coupled to one another via the trans-septal opening. In some embodiments, a mitral valve replacement and a tricuspid valve repair can be coupled to one another via the trans-septal opening.

During ventricular systole, contraction of the ventricles can push the tricuspid valve device and the mitral valve device toward the right atrium and left atrium, respectively, possibly undesirably dislodging the devices. Anchoring the tricuspid valve device and the mitral valve device to the septum wall can facilitate secure positioning of each of the devices at their respective target sites. Tethering the mitral valve device and tricuspid valve device to one another can advantageously balance the forces exerted thereupon by the contraction of the heart. For example, forces exerted upon the tricuspid valve device can be balanced against forces exerted upon the mitral valve device, thereby reducing or eliminating imbalance of forces exerted upon the septum wall. Reducing or eliminating the imbalance of forces exerted upon the septum wall can reduce or prevent damage to the septum wall due to anchoring the tricuspid valve device and the mitral valve device thereto.

Anchoring the tricuspid valve device and mitral valve device to the septum wall can allow for use of valve devices comprising reduced anchoring features on the devices themselves to achieve secure positioning within their respective target positions. Valve devices can have reduced anchoring features for coupling to the native valves to achieve desired anchoring, enabling use of valve devices having reduced sized and/or complexity. For example, a double heart valve anchoring system as described herein can comprise a tricuspid valve replacement and/or a mitral valve replacement which can have a shorter profile and/or a narrower diameter. The tricuspid valve replacement and/or mitral valve replacement can be easier to compress for implantation and/or be simpler to fabricate.

In some embodiments, one or more systems described herein can be advantageously delivered using a minimally invasive transcatheter approach. Both a replacement tricuspid valve and a replacement mitral valve can be delivered using a minimally invasive transcatheter approach, including being positioned in their respective target sites using a single surgical procedure. For example, a delivery catheter carrying both a tricuspid valve replacement and a mitral valve replacement can be inserted into the right atrium (RA) through the inferior vena cava (IVC) or the superior vena cava (SVC), and from the RA into the right ventricle (RV), and subsequently into the left ventricle (LV) via the septal wall. After reaching the target site of the mitral valve replacement, the mitral valve replacement can be released from the delivery catheter. Subsequently, the delivery catheter can be retracted along, or substantially along, the same path along which it was inserted. After the delivery catheter is retracted to the target site for the tricuspid valve replacement, the tricuspid valve replacement can be deployed. Such a procedure can provide a less invasive percutaneous delivery method, reducing trauma to the patient during the surgical procedure, and easing recovery.

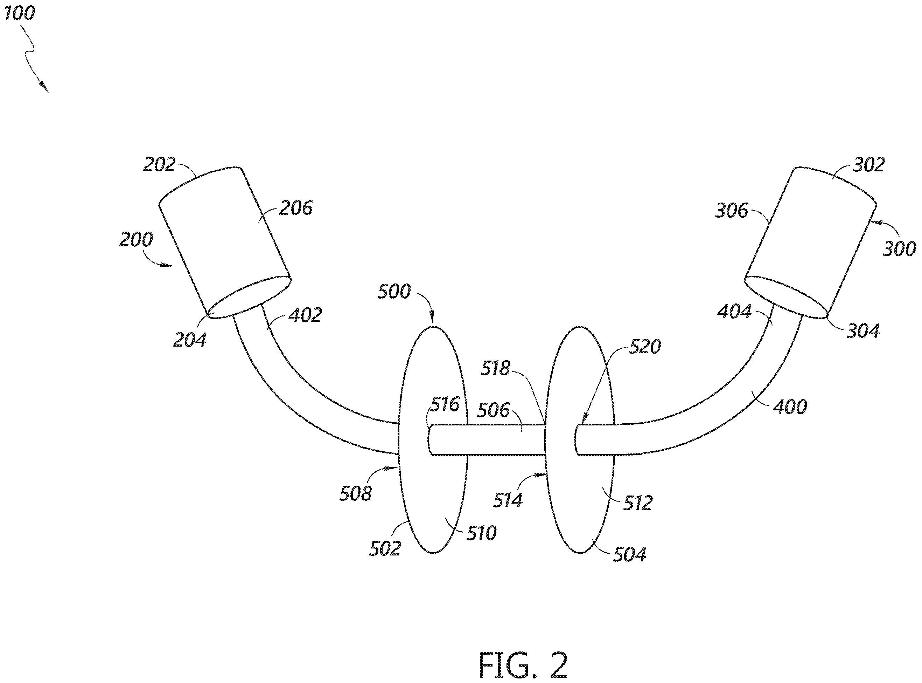

Figure 2 is a schematic diagram of an example of a double heart valve anchoring system 100 comprising two heart valve replacements. In some embodiments, the double heart valve anchoring system 100 can be delivered to its target site using a minimally invasive transcatheter approach. The double heart valve anchoring system 100 can comprise a tricuspid valve replacement 200, a mitral valve replacement 300, and a connector 400 coupling the tricuspid valve replacement 200 and the mitral valve replacement 300 to one another. The connector 400 can be configured to extend through a trans-septal opening formed in an interventricular septum. As described in further detail herein, the connector 400 can be configured to anchor the tricuspid valve replacement 200 and the mitral valve replacement 300 to the septum, while balancing forces exerted by the contraction of the heart upon the tricuspid valve replacement 200 against those exerted by the contraction of the heart upon the mitral valve replacement 300.

Referring again to Figure 2, the tricuspid valve replacement 200 can comprise a tricuspid valve body portion 206 having a first distal end 202 and a second distal 204. The mitral valve replacement 300 can have a mitral valve body portion 306 comprising a first distal end 302 and a second distal end 304. The connector 400 can be coupled to the respective second distal ends 204 and 304 of the tricuspid valve replacement 200 and the mitral valve replacement 300. As described in further detail herein, the connector 400 can be coupled to the ventricular facing ends of the tricuspid valve replacement 200 and the mitral valve replacement 300. For example, the connector 400 can have a first portion 402 coupled to the second distal end 204 of the tricuspid valve replacement 200, and a second portion 404 coupled to the second distal end 304 of the mitral valve replacement 300. Although Figure 2 shows that the connector 400 can be coupled to respective distal ends of the tricuspid valve replacement 200 and the mitral valve replacement 300, it will be understood that the connector 400 can be coupled to one or more other portions of the tricuspid valve replacement 200 and/or the mitral valve replacement 300.

In some embodiments, a connector can be coupled to a distal portion of a heart valve replacement not at a distal end of the heart valve replacement body portion, such as a distal portion proximate to the distal end of the heart valve replacement body portion. In some embodiments, the connector can be coupled to a central portion of the heart valve replacement body portion. In some embodiments, the connector can be coupled to the heart valve replacement at more than one location, including one or more portions of the heart valve replacement as described herein. A location at which the connector couples to the heart valve replacement can be selected to provide a desired angle of anchoring to the septum and/or coupling to another heart valve replacement.

As described in further detail herein, in some embodiments, a length of a connector can be predetermined. For example, the length can be selected prior to insertion of the delivery catheter carrying the double heart valve anchoring system into the patient. In some embodiments, the length can be adjustable during an implantation procedure. For example, the length can be adjusted during the implantation procedure in response to individual anatomy.

In some embodiments, the double heart valve anchoring system 100 can comprise an anchor 500 comprising at least a portion configured to be secured within the trans-septal opening. The anchor 500 can comprise an anchor body 506, and a first septal contact 502 and a second septal contact 504 coupled to opposing distal ends 516, 518 of the anchor body 506. The anchor can comprise a lumen 520 extending along an entire longitudinal length of the anchor body 506.

While the anchor 500 is positioned at its target site, the first distal end 516 of the anchor body 506 can be positioned adjacent or proximate to a right ventricular surface of the septum and the second distal end 518 of the anchor body 506 can be positioned adjacent or proximate to a left ventricular surface of the septum. In some embodiments, a longitudinal length of the anchor body 506 can the same or substantially the same as a thickness of the septum at its target site. The anchor 500 can be positioned at the trans-septal opening such that the lumen 520 extends across the entire thickness of the septum at the opening. The connector 400 can extend through the lumen 520 to couple the tricuspid valve replacement 200 and the mitral valve replacement 300 to one another.

The first septal contact 502 can be configured to be positioned against or proximate to a right ventricular surface of the septum and the second septal contact 504 can be configured to be positioned against or proximate to a left ventricular surface of the septum. As shown in Figure 2, the first septal contact 502 and the second septal contact 504 coupled to respective distal ends 516, 518 of the anchor body 506 can have openings corresponding to the lumen 520 such that the connector 400 can extend therethrough. Extending the connector 400 through the anchor 500 can facilitate stable anchoring of the tricuspid valve replacement 200 and the mitral valve replacement 300 to the septum, while reducing or preventing damage to the septum from the anchoring.

The first septal contact 502 and the second septal contact 504 can be configured to engage the septum to facilitate stable positioning of the anchor 500 in the trans-septal opening. A portion of the septum can be between the first septal contact 502 and the second septal contact 504, for example being sandwiched by the first septal contact 502 and the second septal contact 504. The first septal contact 502 can have a first surface 508 configured to face the right ventricle, and a second surface 510 configured to face the right ventricular surface of the septum. In some embodiments, the second surface 510 can be configured to be positioned adjacent to and in contact with the right ventricular surface. In some embodiments, the second surface 510 can be proximate to but not in contact with the right ventricular surface. The second septal contact 504 can have a first surface 512 configured to face the left ventricle, and a second surface 514 configured to face the left ventricular surface of the septum. In some embodiments, the second surface 514 can be configured to be positioned adjacent to and in contact with the left ventricular surface. In some embodiments, the second surface 514 can be proximate to but not in contact with the left ventricular surface.

Although the first septal contact 504 and the second septal contact 506 described with reference to Figure 2 can comprise a circular or substantially circular shape, and extending around the entire circumference of the lumen 520, it will be understood that a septal contact may extend only partially around the circumference of the lumen 520. In some embodiments, a septal contact can comprise one or more features on a septum facing surface to facilitate secure positioning of the anchor 500, such as one or more protrusions and/or indentations.

The lumen 520 can have any number of cross-sectional shapes. In some embodiments, a cross-sectional shape of the lumen 520 comprises an arcuate shape, including a circle or an oval. In some embodiments, a cross-sectional shape of the lumen 520 comprises a straight edge, such as a rectangle or triangle. A cross-sectional shape of the lumen 520 can be selected based on a cross-sectional shape of the connector 400, for example to accommodate the connector 400. A cross-sectional dimension, such as a diameter, of the lumen 520 can be selected to accommodate the connector 400. As described in further detail herein, a double heart valve anchoring system can comprise multiple connectors. A cross-sectional shape and/or dimension of the lumen 520 can be configured to accommodate the multiple connectors.

In some embodiments, the anchor 500 can facilitate occlusion of the trans-septal opening to prevent or reduce blood flow between the left and right ventricles. For example, the lumen 520 can have a dimension and cross-sectional shape to accommodate the connector 400, without or substantially without permitting flow of blood past the anchor 500 while the anchor 500 is deployed at its target site. In some embodiments, the cross-sectional diameter of the lumen 520 is the same as or similar to that of the connector 400. In some embodiments, the lumen 520 can have a cross-sectional shape the same as or similar to that of the connector 400.

In some embodiments, the anchor 500 and/or the connector 400 can comprise a flexible, deformable and/or elastic biocompatible material configured to facilitate transcatheter delivery to their respective target sites. A material having shape memory can be used. The anchor 500 and/or the connector 400 can comprise one or more of a flexible, deformable and/or elastic biocompatible polymer and/or metal alloy. In some embodiments, the anchor 500 and/or the connector 400 can comprise one or more of polypropylene (PP), polyethylene (PE), polymethylmetacrylate (PMMA), polystyrene (PS), polyvinylchloride (PVC), polytetrafluoroethylene (PTFE), polyurethane (PU), polyamide (nylon), polyethylenterephthalate (PET), polyethersulfone (PES), polyetherimide (PEI), polyetheretherketone (PEEK), polyvinylchloride (PVC), and poly(lactic-co-glycolic) acid (PLGA). In some embodiments, the anchor 500 and/or the connector 400 can comprise stainless steel and/or nickel-titanium. In some embodiments, the connector 400 can comprise a flexible cord. The connector 400 can be a flexible cord. In some embodiments, the connector 400 can comprise a nitinol wire. For example, the connector 400 can be a nitinol wire. In some embodiments, the connector 400 can be a polymeric wire. In some embodiments, the anchor 500 can comprise shape memory material such that the anchor 500 can be advanced through a delivery catheter in a reduced profile configuration. After delivery to the target site, the anchor 500 can be released from the delivery catheter and assume an expanded profile configuration.

In some embodiments, according to the invention a double heart valve anchoring system does not comprise an anchor. For example, a double heart valve anchoring system has a connector extending through the trans-septal opening without being coupled to any anchor positioned in and/or through a septum.

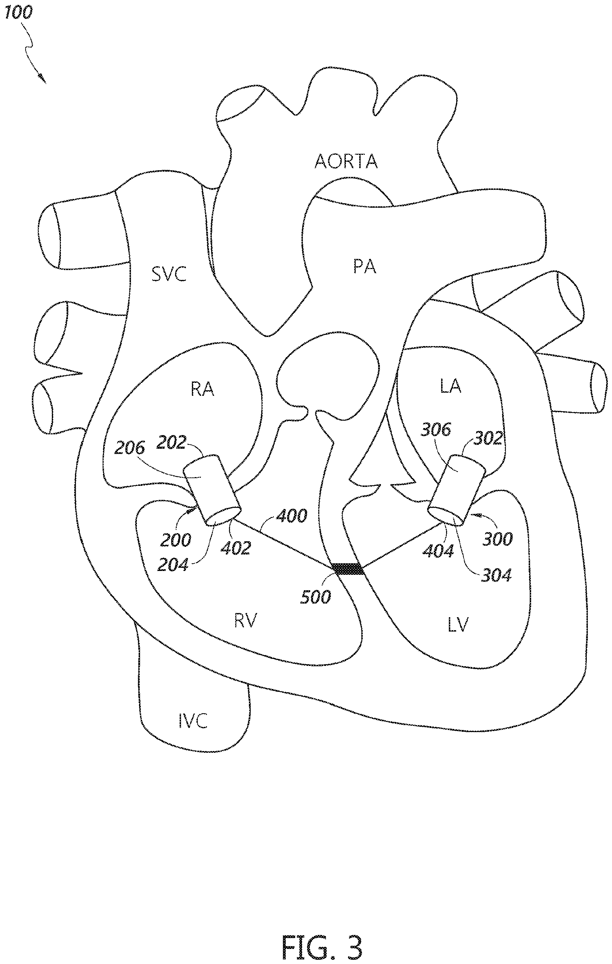

Figure 3 is a schematic diagram showing the double heart valve anchoring system 100 positioned at its target site in the heart. The double heart valve anchoring system 100 can have the tricuspid valve replacement 200 comprising at least a portion positioned within a native tricuspid valve, and the mitral valve replacement 300 comprising at least a portion positioned within a native mitral valve. The connector 400 can couple the tricuspid valve replacement 200 and the mitral valve replacement 300 to one another. The anchor 500 can be positioned at a trans-septal opening in the interventricular septum. The connector 400 can extend through the anchor 500 to couple the tricuspid valve replacement 200 and the mitral valve replacement 300.

A portion of the tricuspid valve body portion 206 can be proximate to and/or in direct contact with one or more parts of the native tricuspid valve. The first distal end 202 of the tricuspid valve replacement 200 can be oriented toward the right atrium and the second distal end 204 can be oriented toward the right ventricle. In some embodiments, the second distal end 204 can be positioned within the right ventricle. In some embodiments, the first distal end 202 can be positioned within the right atrium. A portion of the mitral valve body portion 306 can be proximate to and/or in direct contact with one or more parts of the native mitral valve. The first distal end 302 can be oriented toward the left atrium and the second distal end 304 can be oriented toward the left ventricle. In some embodiments, the second distal end 304 can be positioned within the left ventricle. In some embodiments, the first distal end 302 can be positioned within the left atrium. The first portion 402 of the connector 400 can be coupled to a distal portion of the tricuspid valve replacement 200, such as the second distal end 202 oriented toward the right ventricle, and the second portion 404 of the connector 400 can be coupled to a distal portion of the mitral valve replacement 300, such as the second distal end 302 oriented toward the left ventricle.

During ventricular systole, forces exerted upon the tricuspid valve replacement 200 and the mitral valve replacement 300 by the contractions of the ventricles can push the tricuspid valve replacement 200 and the mitral valve replacement 300 toward the right atrium and the left atrium, respectively. The forces exerted upon the tricuspid valve replacement 200 and the mitral valve replacement 300 can tend to dislodge the tricuspid valve replacement 200 and the mitral valve replacement 300 from their corresponding desired positions within the heart. The connector 400 coupled to the anchor 500 in the septum can provide forces to counter those exerted thereupon by the heart so as to facilitate holding the tricuspid valve replacement 200 and the mitral valve replacement 300 in their respective target positions. A length of the connector 400 can be selected such that the connector 400 is pulled tight while the tricuspid valve replacement 200 and the mitral valve replacement 300 are pushed toward the right atrium and left atrium during systole, holding the tricuspid valve replacement 200 and the mitral valve replacement 300 in place. A length of the connector 400 can be selected such that the connector 400 is taut when the heart is in the systole period. In some embodiments, a length of the connector 400 between the tricuspid valve and the mitral valve can be predetermined. For example, the length of the connector 400 can be selected prior its insertion into a delivery catheter. As described in further detail herein, in some embodiments, the length can be adjustable, for example being determined during an implantation procedure.

The anchor 500 can be positioned at a location along the septum to provide desired anchoring of the double heart valve anchoring system 100 to the septum. For example, the trans-septal opening can be formed at a location along the septum so as to reduce or avoid interference with the chordae tendineae by the anchor 500 and/or connector 400, while providing desired angle of anchoring, and/or selecting an area of the septum which can provide the desired strength. In some embodiments, the trans-septal opening can be formed at or proximate to the mid-point along the septal wall. In some embodiments, the trans-septal opening can be formed above the mid-point along the septal wall.

As described herein, while the heart is in the systole period, the native tricuspid valve and the native mitral valve can exert forces upon the tricuspid valve replacement 200 and the mitral valve replacement 300, respectively, to push the replacement tricuspid valve 200 and replacement mitral valve 300 toward the right atrium and the left atrium. The trans-septal opening can be located at or proximate to a region on the septum where the force vectors of the forces would intersect so as to provide desired angles of anchoring to the septum for the tricuspid valve replacement 200 and the mitral valve replacement 300. In some embodiments, the trans-septal opening can be formed above the intersection point.

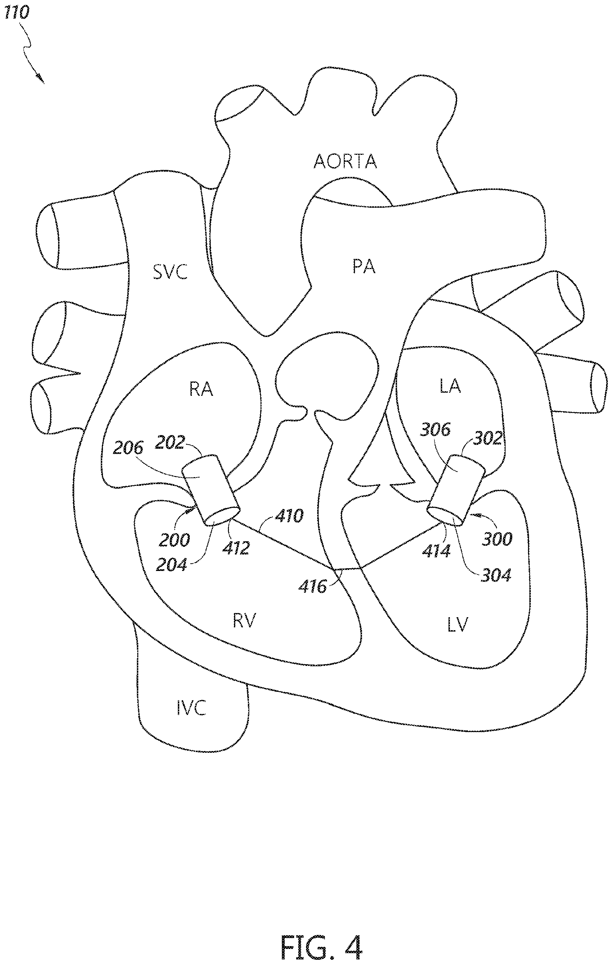

In some embodiments, a double heart valve anchoring system does not include an anchor. Figure 4 is a schematic diagram showing an example of a double heart valve anchoring system 110 positioned at its target site in the heart, where the double heart valve anchoring system 110 does not include an anchor. The double heart valve anchoring system 110 can have a connector 410 coupling the tricuspid valve replacement 200 and the mitral valve replacement 300 to one another. The tricuspid valve replacement 200 can comprise at least a portion positioned within a native tricuspid valve, and the mitral valve replacement 300 can comprise at least a portion positioned within a native mitral valve. The double heart valve anchoring system 110 does not include the anchor 500. As shown in Figure 4, the connector 410 can extend through a trans-septal opening without being coupled to an anchor.

A first portion 412 of the connector 410 can be coupled to the tricuspid valve replacement 200, such as at the second distal end 204. A second portion 414 of the connector 410 can be coupled to the mitral valve replacement 300, such as at the second distal end 304. The connector 410 can comprise a trans-septal portion 416 configured to extend through the trans-septal opening. For example, the trans-septal portion 416 can be in direct contact with the septum.

In some embodiments, the trans-septal portion 416 of the connector 410 can comprise one or more features for engaging with the septum, protecting the septum, and/or occluding the trans-septal opening. The trans-septal portion 416 can engage with the septum and/or have septum protective features to facilitate insertion of the connector 410 through the trans-septal opening. Occlusion of the trans-septal opening can reduce or eliminate blood flow between the left ventricle and the right ventricle. The double heart valve anchoring system 110 can be more compact and/or have a reduced profile, such as compared to a double heart valve anchoring system comprising an anchor. The compact size and/or reduced profile can provide ease of delivery and/or implantation at the target site.

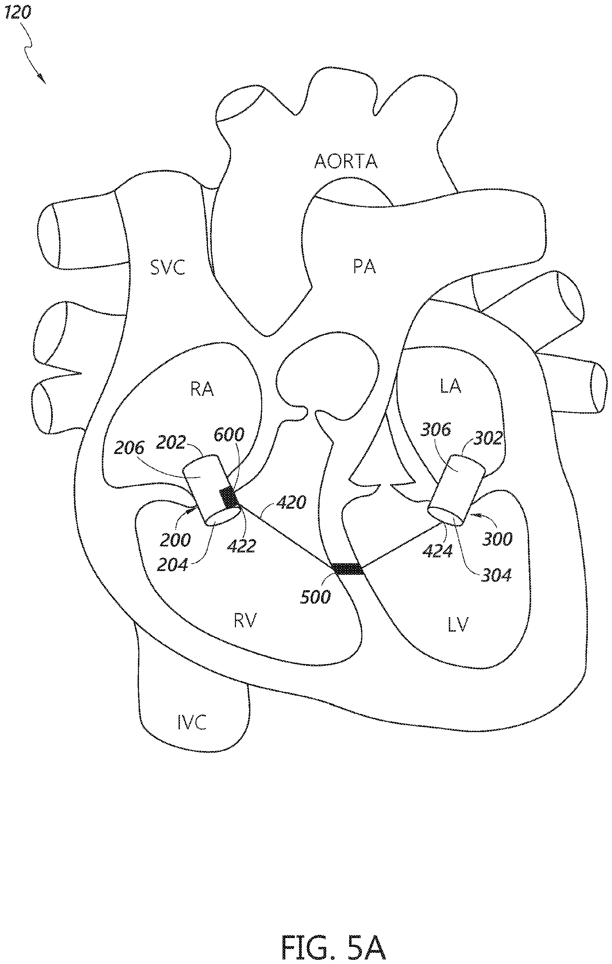

Figure 5A is a schematic diagram of a double heart valve anchoring system 120 comprising an adjustable connector 420. A length of the connector 420 can be adjusted during the implantation procedure. The double heart valve anchoring system 120 is shown in Figure 5A as being positioned at its target location in the heart. The double heart valve anchoring system 120 can comprise the tricuspid valve replacement 200 and the mitral valve replacement 300. The connector 420 can have a first portion 422 coupled to the tricuspid valve replacement 200 and a second portion 424 coupled to the mitral valve replacement 300. The connector 420 can extend through the anchor 500 positioned in a trans-septal opening. The tricuspid valve replacement 200, the mitral valve replacement 300 and the anchor 500 can be positioned in the heart as described herein, such as with reference to Figure 3.

In some embodiments, the length of the connector 420 can be adjusted to accommodate the anatomy of a patient. For example, the length of the connector 420 can be adjusted after the double heart valve anchoring system 120 has been inserted into the patient. As described in further detail herein, the length of the connector 420 can be adjusted one or more times during the implantation procedure, including after the double heart valve anchoring system 120 has been positioned at its target site.

In some embodiments, the double heart valve anchoring system 120 can comprise a locking mechanism 600 configured to secure the length of the connector 420 at the selected length after adjustment has been performed based on the particular anatomy of the individual. The locking mechanism 600 can be deployed, activated, and/or triggered after the appropriate length has been selected, locking the length of the connector 420 at the determined length. In some embodiments, the locking mechanism 600 can be coupled to or integrated as part of the tricuspid valve replacement 300.

Although Figure 5A shows that the double heart valve anchoring system 120 can include one locking mechanism 600, it will be understood that more than one locking mechanism 600 can be included. In some embodiments, one or more of the tricuspid valve replacement 200, mitral valve replacement 300, and anchor 500, can comprise the locking mechanism 600 coupled thereto or integrated therewith.

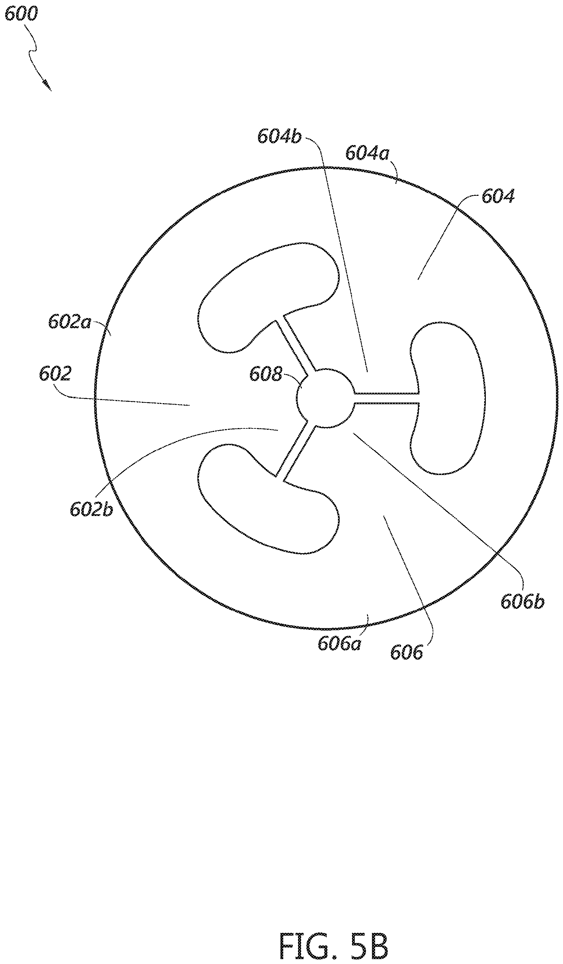

Figure 5B shows a top-down plan view of an example of the locking mechanism 600. The locking mechanism 600 can be in an open state and a closed state. In the open state, the locking mechanism 600 can enable a connector 420 to move freely therethrough. In the closed state, the locking mechanism 600 can reduce or avoid movement of the connector 420 so as to lock the connector 420 at a selected length. Figure 5B shows the locking mechanism 600 in the closed state.

The locking mechanism 600 can comprise a first movable flap 602, a second movable flap 604, and a third movable flap 606, coupled to one another at respective first distal portions 602a, 604a, 606a. A second distal portion 602b, 604b, 606b of the first movable flap 602, second movable flap 604, and third movable flap 606 can define an opening 608. The connector 420 can be extended through the opening 608.

To achieve the open state, the movable flaps 602, 604, 606 can be moved in a first direction to push the second distal portions 602b, 604b, and 606b away from one another to enlarge the opening 608. The connector 420 can move freely through the opening 608 when the locking mechanism is in the open state, for example to enable adjustment of a length of the connector 420 during an implantation procedure. The movable flaps 602, 604, 606 can be configured to be moved in a second direction opposite that of the first direction to move the second distal portions 602b, 604b, and 606b closer together so as to achieve the closed state, reducing the size of the opening 608. One or more edges of the second distal portions 602b, 604b, and 606b defining the opening 608 can be configured to sufficiently contact the connector 420 while the locking mechanism 600 is in the closed state so as to reduce or prevent movement of the connector 420 through the opening 608, thereby facilitating locking or substantially locking the connector 420 at an adjusted length. For example, the opening 608 can be the same size or a smaller size than a cross-sectional area of the connector 420 in the closed state such that one or more edges of the second distal portions 602b, 604b, and 606b can sufficiently contact the connector 420 to reduce or prevent movement of the connector 420 therethrough.

In some embodiments, the locking mechanism 600 can have a non-planar configuration. For example, the movable flaps 602, 604, 606 can be oriented at an angle relative to one another, extending into the page of Figure 5B, such that movement of the flaps 602, 604, 606 in the first direction (e.g., into the page) pushes the second distal portions 602b, 604b, 606b further apart to achieve the open state. Movement of the flaps 602, 604, 606 in the second direction (e.g., out of the page) can bring the second distal portions 602b, 604b, 606b closer together to achieve the closed state.

Opening and closing the locking mechanism 600 can be controlled using various techniques. In some embodiments, opening and closing the locking mechanism 600 can be triggered by pressure. For example, application of pressure can be used to move the movable flaps 602, 604, and 606 to open and/or close the locking mechanism. In some embodiments, contacting the connector 420 to the movable flaps 602, 604, and 606 can trigger opening and/or closing of the locking mechanism 600. For example, contacting the connector 420 to the movable flaps 602, 604, and 606 and moving the connector 420 relative to the movable flaps 602, 604, and 606 can cause the movable flaps 602, 604, and 606 to move in the desired direction. Moving the connector 420 in the first direction while contacting the connector 420 and the movable flaps 602, 604, and 606 can open the locking mechanism 600, and moving the connector 420 in the second direction while contacting the connector 420 and the movable flaps 602, 604, and 606 can close the locking mechanism 600. In some embodiments, the connector 420 can comprise one or more features, such as protrusions and/or indentations, to facilitate engagement with the movable flaps 602, 604, and 606.

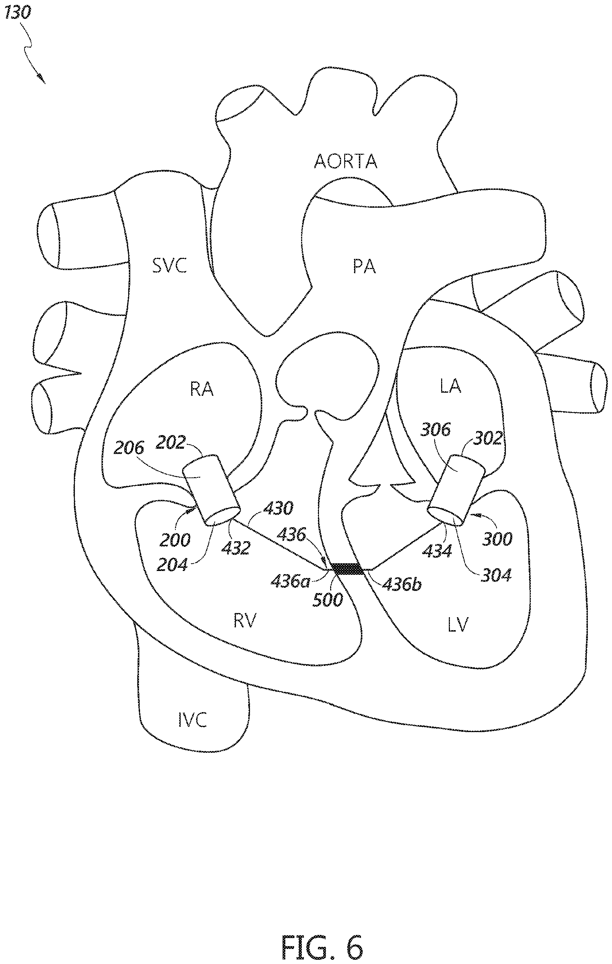

Figure 6 is a schematic diagram of a double heart valve anchoring system 130 which includes a connector 430 comprising a rigid or semi-rigid portion 436. The double heart valve anchoring system 130 is shown as being positioned at its target site in the heart. The tricuspid valve replacement 200, the mitral valve replacement 300, and the anchor 500 can be positioned in their respective target positions, such as described with reference to Figure 3. The connector 430 can extend through the anchor 500 configured to be positioned in a trans-septal opening, and couple the tricuspid valve replacement 200 and the mitral valve replacement 300 to one another. The connector 430 can have a first portion 432 coupled to the tricuspid valve replacement 200 and a second portion 434 coupled to the mitral valve replacement 300.

The rigid or semi-rigid portion 436 of the connector 430 can be configured to extend through the trans-septal opening, for example extending through the anchor 500 configured to be positioned in the trans-septal opening. The rigid or semi-rigid portion 436 can extend into one or both of the right ventricle and the left ventricle. For example, the rigid or semi-rigid portion 436 can comprise a first segment 436a configured to extend into the right ventricle and a second segment 436b configured to extend into at the left ventricle. A longitudinal length of the first segment 436a and/or the second segment 436b can be selected to provide desired anchoring of the tricuspid valve replacement 200 and the mitral valve replacement 300 to the septum. In some embodiments, the length of the first segment 436a and/or the second segment 436b can be selected so as to provide predetermined angles of tethering of the tricuspid valve replacement 200 and the mitral valve replacement 300 to the septum. The length of the first segment 436a and/or the second segment 436b can be selected so as to enable positioning the trans-septal opening at a location on the septum to reduce or avoid interference with the chordae tendineae while providing desired anchoring of tricuspid valve replacement 200 and the mitral valve replacement 300 to the septum.

In some embodiments, a longitudinal length of the first segment 436a can be the same as or similar to that of the second segment 436b. In some embodiments, a longitudinal length of the first segment 436a is different from that of the second segment 436b. The longitudinal length of the first segment 436a and/or second segment 436b can be predetermined or adjustable. For example, the longitudinal length of the first segment 436a and/or second segment 436b can be selected prior to inserting the double heart valve anchoring system 130 into the patient. In some embodiments, the length of the first segment 436a and/or the second segment 436b can be adjusted during the implantation procedure based on individual anatomy to provide the desired anchoring. In some embodiments, the length of the first segment 436a and/or the second segment 436b can be adjusted after insertion of the double heart valve anchoring system 130 into the patient. For example, the length of the first segment 436a and the second segment 436b length can be adjusted by moving the rigid or semi-rigid portion 436 forward or backward within the lumen 520 of the anchor 500. In some embodiments, the rigid or semi-rigid portion 436 can be translated distally to increase the length of the second segment 436b, while decreasing the length of the first segment 436a. In some embodiments, the rigid or semi-rigid portion 436 can be translated proximally to increase the length of the first segment 436a, while decreasing the length of the second segment 436b.

The connector 430 can comprise one or more biocompatible materials described herein, including a polymeric and/or metallic material. In some embodiments, the rigid or semi-rigid portion 436 of the connector 430 can comprise a material different from the remainder of the connector 430. In some embodiments, the rigid or semi-rigid portion 436 of the connector 430 can comprise the same material as the remainder of the connector 430.

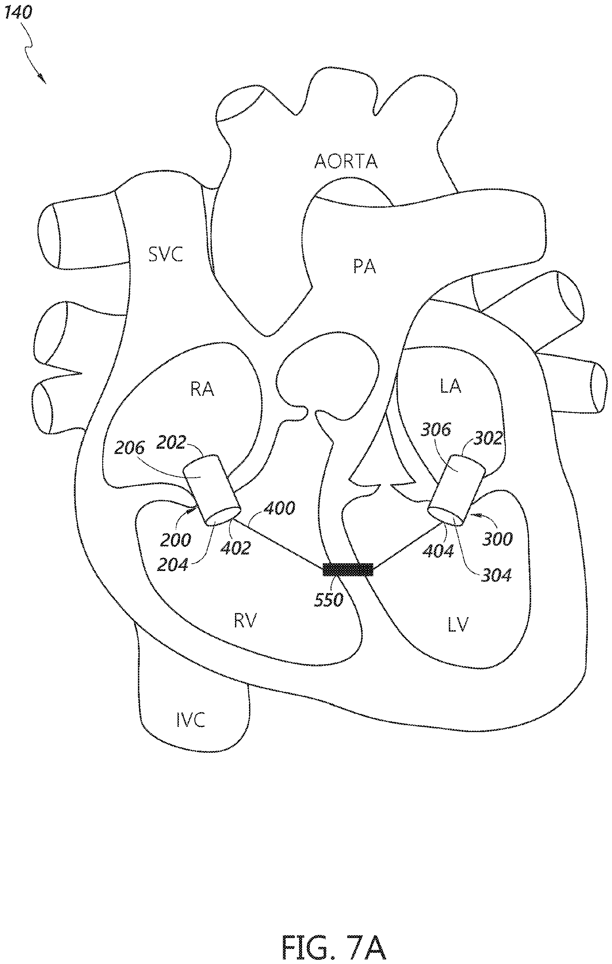

Figure 7A is a schematic diagram of an example of a double heart valve anchoring system 140 which includes an anchor 550 comprising respective rigid or semi-rigid portions configured to extend into the right ventricle and the left ventricle. The double heart valve anchoring system 140 can comprise the tricuspid valve replacement 200 and the mitral valve replacement 300, and the connector 400 coupling the tricuspid valve replacement 200 and the mitral valve replacement 300 to one another. The connector 400 can extend through the anchor 550 configured to be positioned in a trans-septal opening. The tricuspid valve replacement 200 and the mitral valve replacement 300 can be positioned in the heart, such as described with reference to Figure 3.

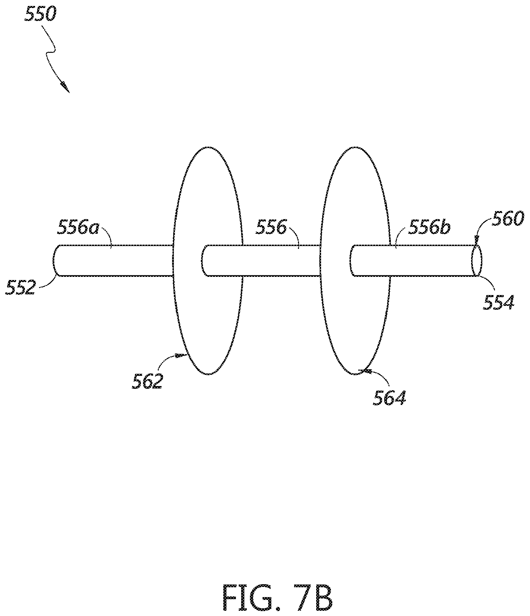

Figure 7B is a schematic diagram of the anchor 550. The anchor 550 can comprise an anchor body portion 556 comprising a first rigid or semi-rigid portion 556a configured to extend into the right ventricle and a second rigid or semi-rigid portion 556b configured to extend into the left ventricle. For example, the anchor body portion 556 can comprise a first distal end 552 which extends into the right ventricle and a second distal end 554 which extends into the left ventricle. The first rigid or semi-rigid portion 556a can extend into the right ventricle such that the first distal end 552 is positioned in the right ventricle and the second rigid or semi-rigid portion 556b can extend into the left ventricle such that the second distal end 554 is positioned in the left ventricle. The anchor 550 can comprise a lumen 560 which extends along the entire longitudinal length of the anchor body portion 556 such that the connector 400 can be inserted therethrough to couple the tricuspid valve replacement 200 and the mitral valve replacement 300 to one another while the anchor 550 is positioned in the trans-septal opening.

The anchor can include a first septal contact 562 and a second septal contact 564 extending from the anchor body portion 556 and spaced apart so as to engage with the septum. In some embodiments, the first septal contact 562 and the second septal contact 564 can comprise one or more features of the first septal contact 502 and the second septal contact 504 described with reference to Figure 2.

The rigid or semi-rigid portions 556a, 556b of the anchor 500 can be configured to extend into the right ventricle and the left ventricle, respectively, to facilitate desired anchoring of the tricuspid valve replacement 200 and the mitral valve replacement 300 to the septum. In some embodiments, a longitudinal dimension of the first rigid or semi-rigid portion 556a and/or a longitudinal dimension of the second rigid or semi-rigid portion 556b can be selected such that the tricuspid valve replacement 200 and the mitral valve replacement 300 can be properly anchored to the septum, without or substantially without interfering with the chordae tendineae.

In some embodiments, the longitudinal length of the first rigid or semi-rigid portion 556a and the longitudinal length of the second rigid or semi-rigid portion 556b can be the same or similar. In some embodiments, the longitudinal length of the first rigid or semi-rigid portion 556a and the longitudinal length of the second rigid or semi-rigid portion 556b can be different. In some embodiments, the anchor 500 can comprise either the first rigid or semi-rigid portion 556a or the second rigid or semi-rigid portion 556b, but not both.

The anchor 550 can comprise one or more biocompatible materials described herein, including a polymeric and/or metallic material. In some embodiments, the rigid or semi-rigid portions 556a, 556b of the anchor 550 can comprise a material different from the remainder of the anchor 550. In some embodiments, the rigid or semi-rigid portions 556a, 556b of the anchor 550 can comprise the same material as the remainder of the anchor 550.

In some embodiments, the double heart valve anchoring system 140 can comprise one or more other features as described herein. In some embodiments, the double heart valve anchoring system 140 can comprise a plurality of connectors 400. In some embodiments, the double heart valve anchoring system 140 can comprise one or more adjustable connectors 420. In some embodiments, the double heart valve anchoring system 140 can comprise one or more of the connectors 430 comprising a rigid or semi-rigid portion 436. For example, a plurality of connectors can extend through the anchor 550. Respective distal ends of the plurality of connectors can be coupled to corresponding positions on the respective distal ends of the tricuspid valve replacement 200 and the mitral valve replacement 300.

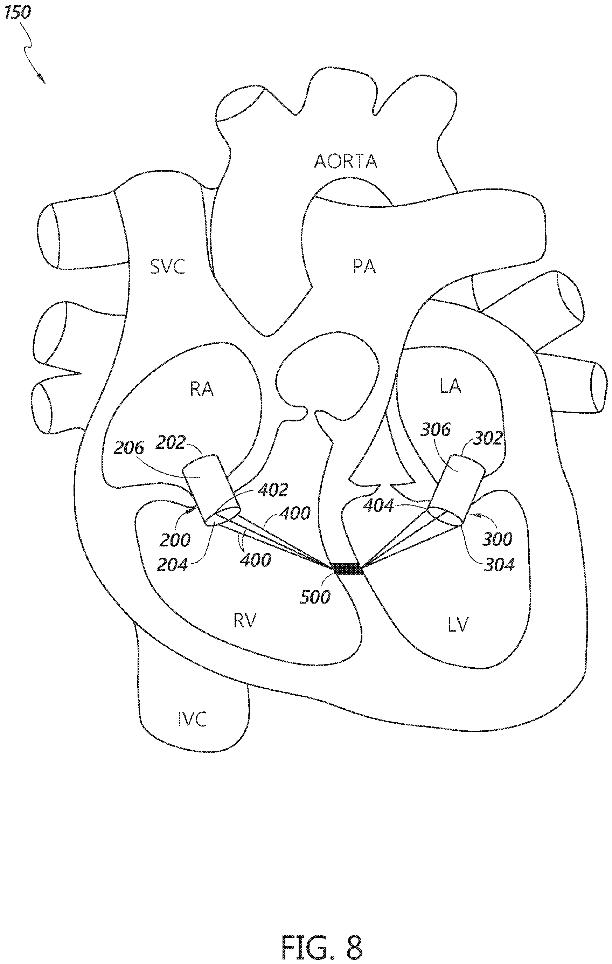

In some embodiments, a double heart valve anchoring system can comprise a plurality of connectors, where the plurality of connectors extend through the same anchor. Figure 8 is a schematic diagram of an example of a double heart valve anchoring system 150 comprising three connectors 400. The double heart valve anchoring system 150 is shown as being positioned at its target site. The double heart valve anchoring system 150 can comprise the tricuspid valve replacement 200, the mitral valve replacement 300, and the anchor 500 positioned at their respective target sites, such as described with reference to Figure 3. The three connectors 400 can couple the tricuspid valve replacement 200 and the mitral valve replacement 300 to one another.

In some embodiments, corresponding ends of each of the three connectors 400 can be coupled to respective distal ends of the tricuspid valve replacement 200 and the mitral valve replacement 300. For example, a first portion 402 of each of the three connectors 400 can be coupled to a second distal end 204 of the tricuspid valve replacement 200 oriented toward the right ventricle. The second portion 404 of each of the three connectors 400 can be coupled to a second distal end 304 of the mitral valve replacement 300 oriented toward the left ventricle. The locations on the second distal end 204 of the tricuspid valve replacement 200 and the second distal end 304 of the mitral valve replacement 300 at which the connectors 400 are coupled can be selected to provide desired anchoring to the septum. In some embodiments, the locations at which the connectors 400 are coupled to the tricuspid valve replacement 200 and/or the mitral valve replacement 300 can be evenly distributed across the corresponding second distal ends 204, 304. For example, the locations at which the connectors 400 are coupled to the second distal ends 204, 304 can be evenly distributed around a perimeter of the second distal ends 204, 304.

The three connectors 400 can extend through the same trans-septal opening. For example, the three connectors 400 can each comprise a corresponding portion which extends through the anchor 500 positioned in the trans-septal opening. In some embodiments, a length for one or more of the three connectors 400 can be predetermined, such as being selected prior to their insertion into a patient.

In some embodiments, the double heart valve anchoring system 150 can comprise one or more other features as described herein. In some embodiments, the double heart valve anchoring system 150 can comprise the anchor 550 rather than the anchor 500. In some embodiments, the double heart valve anchoring system 150 can comprise one or more of the connectors 430 comprising a rigid or semi-rigid portion 436. In some embodiments, the double heart valve anchoring system 150 can comprise one or more adjustable connectors 420. In some embodiments, a length for one or more of the three connectors can be adjusted during an implantation procedure independent of the other connectors. For example, the three connectors may not be coupled to one another within the anchor such that each connector can be moved relative to the others within the lumen of the anchor. In some embodiments, corresponding portions of the three connectors extending through the anchor are coupled to one another. In some embodiments, the double heart valve anchoring system 150 can comprise one or more locking mechanisms 600 as described herein to secure the length of the connector after a desired length is selected.

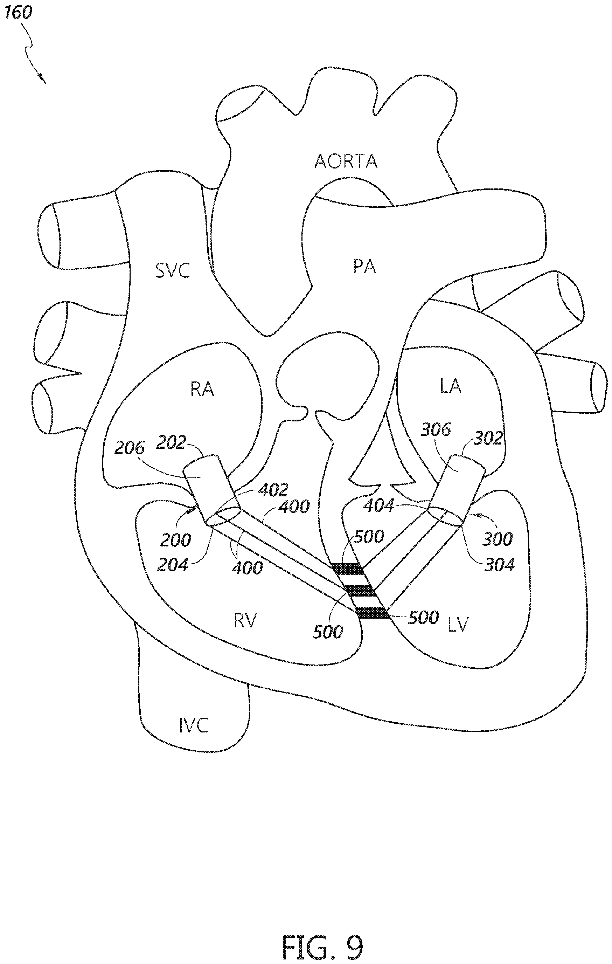

In some embodiments, a double heart valve anchoring system can comprise a plurality of connectors, where the plurality of connectors each extend through a corresponding anchor. Figure 9 is a schematic diagram of an example of a double heart valve anchoring system 160 comprising three connectors 400 and three anchors 500. The double heart valve anchoring system 160 is shown as being positioned at its target site. The double heart valve anchoring system 160 can comprise the tricuspid valve replacement 200 and the mitral valve replacement 300. The tricuspid valve replacement 200 and the mitral valve replacement 300 can be positioned at their respective target sites, such as described with reference to Figure 3. The three connectors 400 can couple the tricuspid valve replacement 200 and the mitral valve replacement 300 to one another. Each of the three connectors 400 can extend through a corresponding anchor 500 positioned in the septum.

Corresponding portions of each of the three connectors 400 can be coupled to respective distal ends of the tricuspid valve replacement 200 and the mitral valve replacement 300. For example, a first portion 402 of each of the three connectors 400 can be coupled to a second distal end 204 of the tricuspid valve replacement 200 and the second portion 404 of each of the three connectors 400 can be coupled to a second distal end 304 of the mitral valve replacement 300. In some embodiments, the locations at which the connectors 400 are coupled to the tricuspid valve replacement 200 and/or the mitral valve replacement 300 can be evenly distributed across the corresponding second distal ends 204, 304. For example, the locations at which the connectors 400 are coupled to the second distal ends 204, 304 can be evenly distributed around a perimeter of the second distal ends 204, 304. The locations on the second distal end 204 of the tricuspid valve replacement 200 and the second distal end 304 of the mitral valve replacement 300 at which the connectors 400 are coupled can be selected to provide desired anchoring to the septum.

The location of the trans-septal openings for positioning the three anchors 500 can be selected to provide desired anchoring. Figure 9 shows the three anchors 500 as being arranged at three different locations along a vertical dimension of the septum. In some embodiments, at least some of the three anchors 500 can be positioned along the same vertical position on the septum. In some embodiments, at least some of the three anchors 500 can be positioned along the same lateral position on the septum. In some embodiments, each of the three anchors 500 can be positioned at different lateral and vertical positions on the septum.

In some embodiments, the double heart valve anchoring system 160 can comprise one or more other features as described herein. In some embodiments, the double heart valve anchoring system 160 can comprise one or more of the anchor 550. In some embodiments, the double heart valve anchoring system 150 can comprise one or more of the connectors 430 comprising a rigid or semi-rigid portion 436. In some embodiments, the double heart valve anchoring system 150 can comprise one or more adjustable connectors 420. For example, the double heart valve anchoring system 160 can comprise one or more locking mechanisms as described herein to secure the length of the connector after a desired length is selected.

In some embodiments, two connectors can extend through the same anchor. Although the double heart valve anchoring system 150 described with reference to Figure 8 comprises three connectors, and the double heart valve anchoring system 160 described with reference to Figure 9 comprises three connectors, it will be understood that a double heart valve anchoring system can comprise more or fewer connectors.

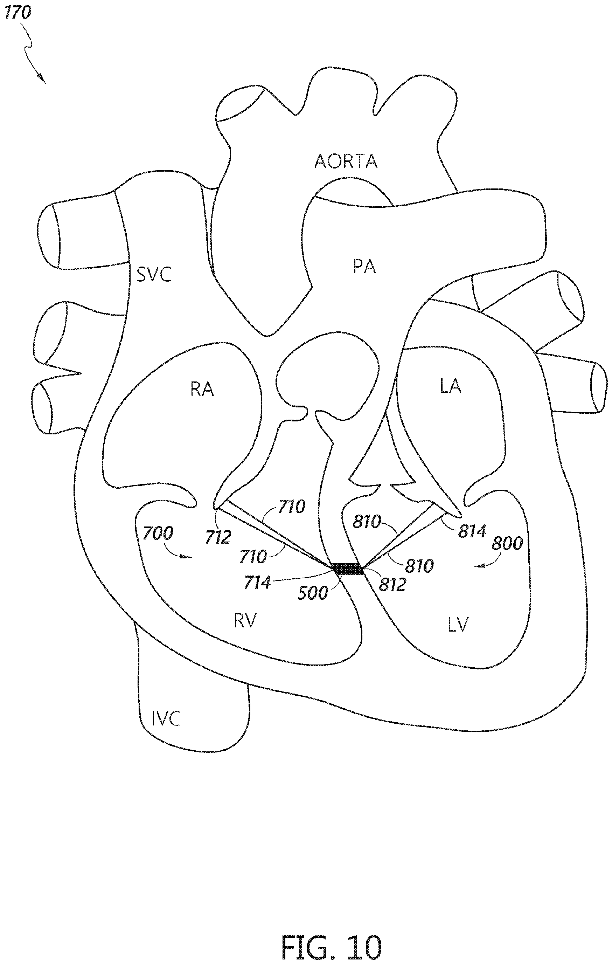

In some embodiments, a double heart valve anchoring system can comprise a tricuspid valve repair and a mitral valve repair anchored to the septum. Figure 10 is a schematic diagram of an example of a double heart valve anchoring system 170 comprising a tricuspid valve repair 700 and a mitral valve repair 800. The tricuspid valve repair 700 and the mitral valve repair 800 can be coupled to one another and be configured to be anchored to the septum. For example, the tricuspid valve repair 700 and the mitral valve repair 800 can be coupled to the anchor 500 configured to be positioned in a trans-septal opening. The tricuspid valve repair 700 can comprise a plurality of connectors 710 configured to couple a native tricuspid valve leaflet to the anchor 500 positioned in the trans-septal opening. A first distal portion 712 of each connector 710 can be coupled to the native tricuspid valve leaflet and a second distal portion 714 can be coupled to the anchor 500. The mitral valve repair 800 can comprise a plurality of connectors 810 configured to couple a native mitral valve leaflet to the anchor 500 positioned in the trans-septal opening. For example, a first distal portion 814 of each connector 810 can be coupled to the native mitral valve leaflet and a second distal portion 812 can be coupled to the anchor 500. Figure 10 shows that the tricuspid valve repair 700 can include two connectors 710 and the mitral valve repair 800 can include two connectors 810, although it should be understood that more or fewer connectors 710, 810 can be used.

In some embodiments, a connector 710 and a connector 810 can be one connector, such that the one connector is configured to extend through the anchor 500 and couple the native tricuspid valve leaflet and the native mitral valve leaflet to one another. For example, each of the plurality of connectors 710 and a corresponding one of the plurality of connectors 810 can be the same connector. In some embodiments, none of the plurality of connectors 710 and the plurality of connectors 810 are the same connector.

In some embodiments, a double heart valve anchoring system can comprise a heart valve replacement and a heart valve repair. For example, a double heart valve anchoring system can comprise a tricuspid valve replacement and a mitral valve repair. Alternatively, a double heart valve anchoring system can comprise a tricuspid valve repair and a mitral valve replacement.

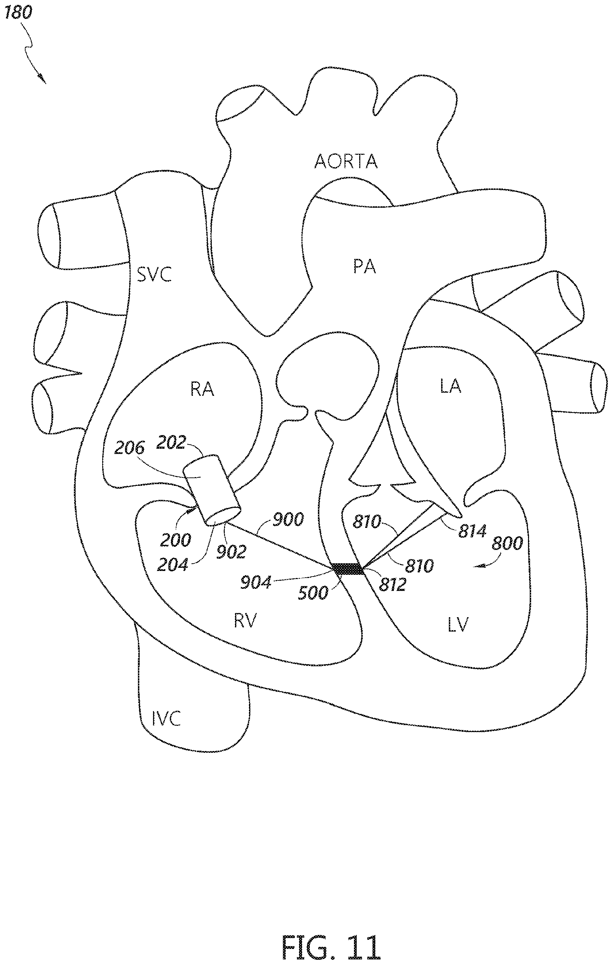

Figure 11 is a schematic diagram of an example of a double heart valve anchoring system 180 comprising the tricuspid valve replacement 200 and the mitral valve repair 800 coupled to one another via the septum. The tricuspid valve replacement 200 and the mitral valve repair 800 can be coupled to the anchor 500 configured to be positioned in a trans-septal opening. The mitral valve repair 800 can comprise the connectors 810 configured to couple a native mitral valve leaflet to the anchor 500. A connector 900 can couple the tricuspid valve replacement 200 to the anchor 500. For example, a first end 902 of the connector 900 can be coupled to the tricuspid valve replacement 200, such as a second distal end 204 of the tricuspid valve replacement 200. A second end 904 of the connector 900 can be coupled to the anchor 500.

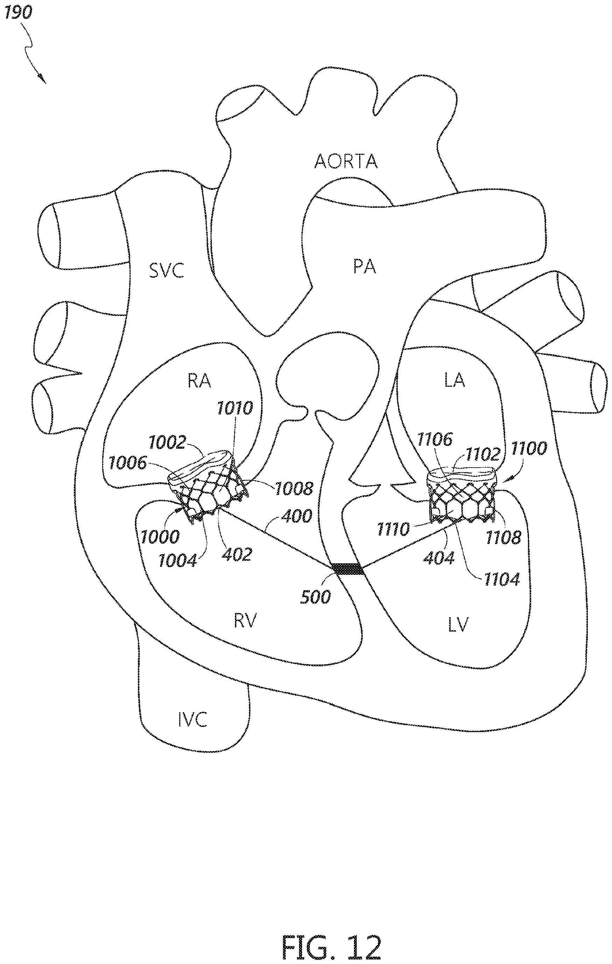

Referring to Figure 12, a schematic diagram is shown of a double heart valve anchoring system 190 comprising an example of a tricuspid valve replacement 1000 and an example of mitral valve replacement 1100. A connector 400 can couple the tricuspid valve replacement 1000 and the mitral valve replacement 1100 to one another. A corresponding portion of the connector 400 can extend through the anchor 500 configured to be positioned in a trans-septal opening.

The tricuspid valve replacement 1000 can comprise a tricuspid valve body portion 1006 comprising a first distal end 1002 and a second distal end 1004. The mitral valve replacement 1100 can comprise a mitral valve body portion 1106 comprising a first distal end 1102 and a second distal end 1104. As shown in Figure 12, the first distal end 1002 of the tricuspid valve replacement 1000, and the first distal end 1102 of the mitral valve replacement 1100 can be oriented towards the right atrium and left atrium, respectively. The second distal ends 1004, 1104 can be oriented toward the right ventricle and left ventricle, respectively. The tricuspid valve replacement 1000 can comprise a frame 1008 and valve tissue 1010. The mitral valve replacement 1100 can comprise a frame 1108 and valve tissue 1110. In some embodiments, a first portion 402 of the connector 400 can be coupled to the second distal end 1004 of the tricuspid valve replacement 1000 and a second portion 404 of the connector 400 can be coupled to the second distal end 1104 of the mitral valve replacement 1100. In some embodiments, the connector 400 can be coupled to the frames 1008, 1108 of the tricuspid valve replacement 1000 and the mitral valve replacement 1100 at their respective second distal ends 1004, 1104. For example, the frames 1008, 1108 can comprise one or more features to facilitate attachment of the connector 400 thereto.

In some embodiments, the double heart valve anchoring system 190 can comprise one or more other features as described herein. In some embodiments, the double heart valve anchoring system 190 can comprise a plurality of connectors 400. In some embodiments, the double heart valve anchoring system 190 can comprise one or more adjustable connectors 420. In some embodiments, the double heart valve anchoring system 190 can comprise one or more of the connectors 430 comprising a rigid or semi-rigid portion 436. In some embodiments, the double heart valve anchoring system 190 can comprise the anchor 550 rather than the anchor 500.

In some embodiments, one or more heart valve replacements described herein can be one or more commercially available heart valve replacements (e.g., Sapien 3^{™}, CardiAQ ^{™}, and Centera ^{™}, commercially available from Edwards Lifesciences LLC, Irvine, CA). In some embodiments, one or more of the double heart valve anchoring systems as described herein may not comprise an anchor. For example, the connector(s) described with reference to Figures 5A, 6, 7A, 8, 9, 10, 11 and 12 can extend through the trans-septal opening without extending through any anchors.

A procedure for positioning a double heart valve anchoring system at its target site can comprise inserting and retracting a double heart valve anchoring system delivery catheter along the same or similar path. Using one path for both the insertion and retraction of the delivery catheter to deploy both a tricuspid valve replacement and a mitral valve replacement anchored to the septum can, for example, enable a simpler minimally invasive transcatheter approach, reduce trauma to the patient, and/or facilitate an improved patient recovery process.

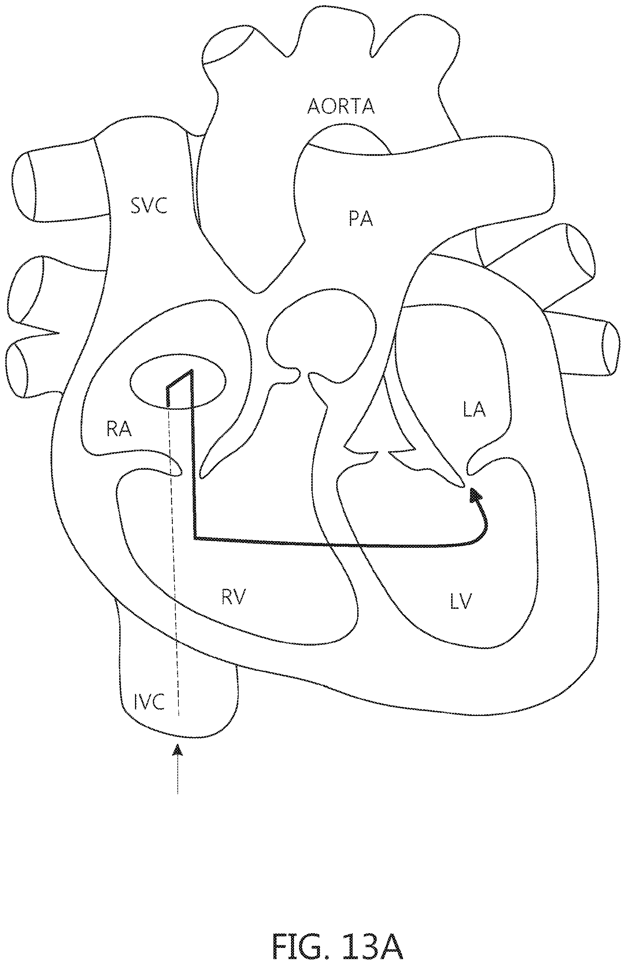

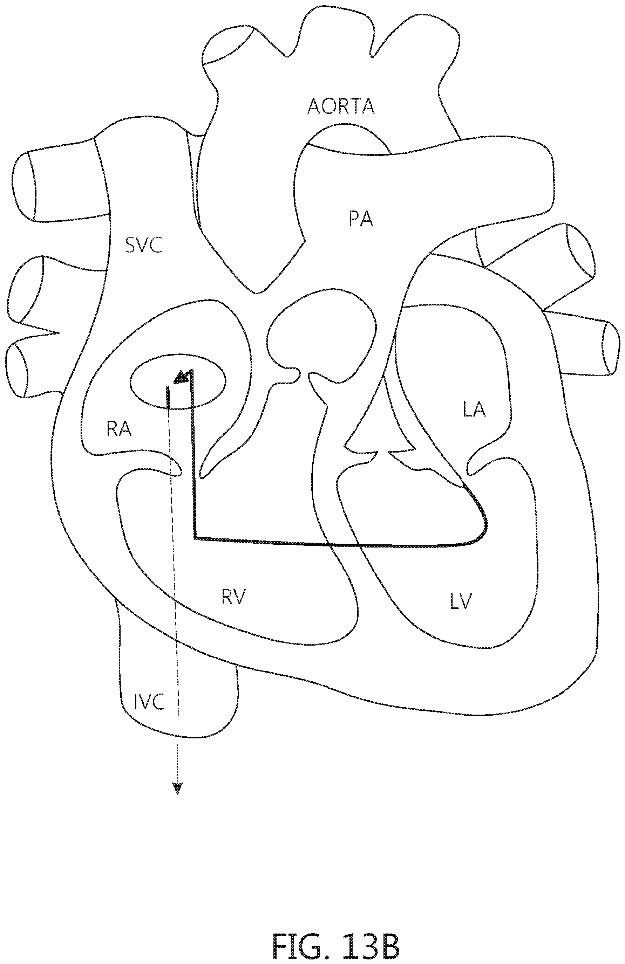

Figures 13A and 13B are schematic diagrams showing an example of a path along which a delivery catheter carrying a double heart valve anchoring system can be advanced and retracted to position the double heart valve anchoring system at its target site. The double heart valve anchoring system can have one or more features described herein (e.g., the double heart valve anchoring systems 100, 110, 120, 130, 140, 150, 190). For example, the double heart valve anchoring system can comprise a tricuspid valve replacement and a mitral valve replacement, and a connector coupling the tricuspid valve replacement and the mitral valve replacement to one another. The connector can extend through an anchor configured to be positioned in a trans-septal opening. Figure 13A shows the path along which the delivery catheter is inserted, and Figure 13B shows the path along which the delivery catheter is retracted.

Referring to Figure 13A, the delivery catheter carrying the double heart valve anchoring system can be advanced through the inferior vena cava (IVC). For example, a trans-femoral approach can be used, inserting the delivery catheter through the femoral vein to the inferior vena cava. A distal end of the delivery catheter can be extended from the inferior vena cava into the right atrium (RA). The distal end of the delivery catheter can then be inserted through an opening formed by the native tricuspid valve and advanced from the right atrium into the right ventricle (RV). Subsequently, the delivery catheter can be extended into the left ventricle (LV) from the right ventricle via a trans-septal opening formed in the septum. After the delivery catheter is inserted into the left ventricle, the distal end of the delivery catheter can be positioned at the target site for the mitral valve replacement and the mitral valve replacement can be deployed at its target position.

Referring to Figure 13B, after the mitral valve replacement is positioned at its target site, the delivery catheter can be retracted towards the right ventricle. The delivery catheter can be retracted along the same or substantially the same path along which the delivery catheter was advanced. As the delivery catheter is retracted through the left ventricle towards the right ventricle, a corresponding portion of the connector can be released in the left ventricle from the distal end of the delivery catheter. The distal end of the delivery catheter can then be withdrawn through the trans-septal opening from the left ventricle back into the right ventricle (RV), leaving the anchor at its target site. At least a portion of the anchor and a corresponding portion of the connector can be positioned in the trans-septal opening as the delivery catheter is moved back through the trans-septal opening. Subsequently, a corresponding portion of the connector can be released in the right ventricle as the distal end of the delivery catheter is retracted through the right ventricle toward the right atrium. The distal end of the delivery catheter can then be positioned at a desired location for the tricuspid valve replacement. After positioning the distal end of the delivery catheter at the desired location for the tricuspid valve replacement, the tricuspid valve replacement can be deployed at its target site. The delivery catheter can then be withdrawn into the right atrium, and removed through the inferior vena cava.

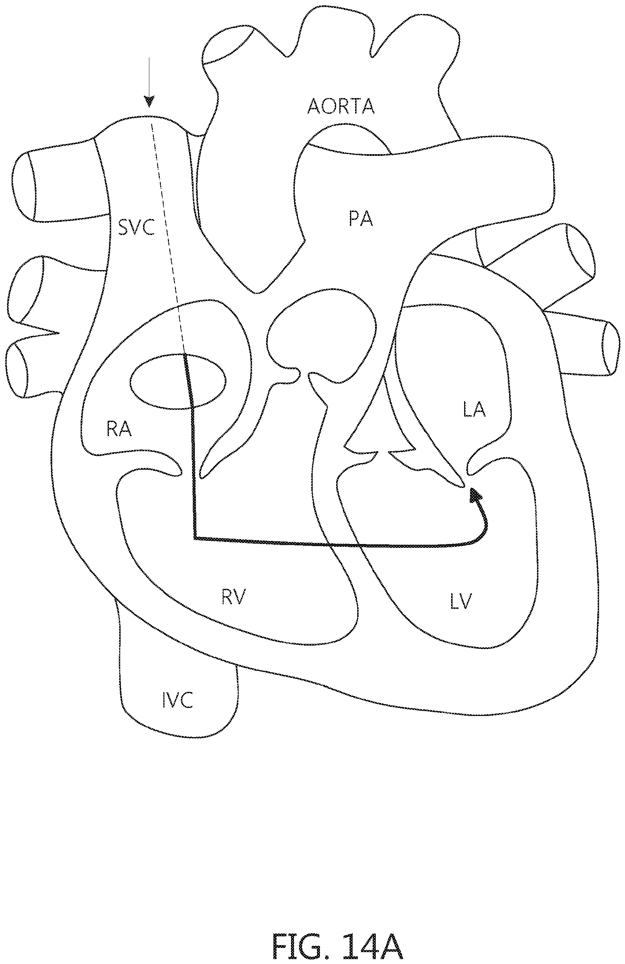

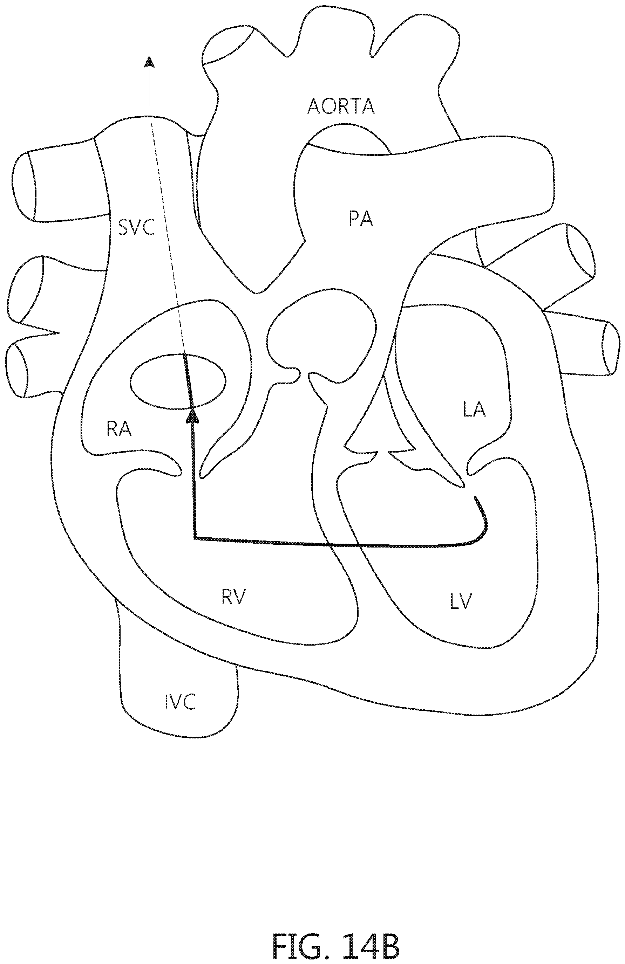

Figures 14A and 14B are schematic diagrams showing another example of a path along which a delivery catheter carrying a double heart valve anchoring system can be advanced and retracted to position the double heart valve anchoring system at its target site. The double heart valve anchoring system can have one or more features described herein (e.g., the double heart valve anchoring systems 100, 110, 120, 130, 140, 150, 190), such as a connector coupling a tricuspid valve replacement and a mitral valve replacement, and the connector extending through an anchor. Figure 14A shows the path along which the delivery catheter can be inserted, and Figure 14B shows the path along which the delivery catheter can be retracted.

Referring to Figure 14A, the delivery catheter carrying the double heart valve anchoring system can be advanced through the superior vena cava (SVC). For example, a trans-subclavian or trans-jugular approach can be used, inserting the delivery catheter through the subclavian vein or the jugular vein to the superior vena cava. A distal end of the delivery catheter can be extended from the superior vena cava into the right atrium (RA), and then from the right atrium through an opening formed by the native tricuspid valve into the right ventricle (RV). Subsequently, the delivery catheter can be extended into the left ventricle (LV) from the right ventricle via a trans-septal opening formed in the septum. After the delivery catheter is inserted into the left ventricle, the distal end of the delivery catheter can be positioned at the target site for the mitral valve replacement. The mitral valve replacement can then be deployed at its target position.

Referring to Figure 14B, after deployment of the mitral valve replacement, the delivery catheter can be retracted towards the right ventricle. The delivery catheter can be retracted along the same or substantially the same path along which the delivery catheter was advanced. As the delivery catheter is retracted through the left ventricle towards the right ventricle, a corresponding portion of the connector can be released in the left ventricle from the distal end of the delivery catheter. The distal end of the delivery catheter can then be withdrawn through the trans-septal opening into the right ventricle (RV). The anchor can be released at its target site in the trans-septal opening, along with a corresponding portion of the connector. Subsequently, a corresponding portion of the connector can be released in the right ventricle as the distal end of the delivery catheter is retracted through the right ventricle toward the right atrium. The distal end of the delivery catheter can then be positioned at a desired location for the tricuspid valve replacement, and the tricuspid valve replacement can be deployed. The delivery catheter can then be withdrawn into the right atrium, and removed through the superior vena cava.

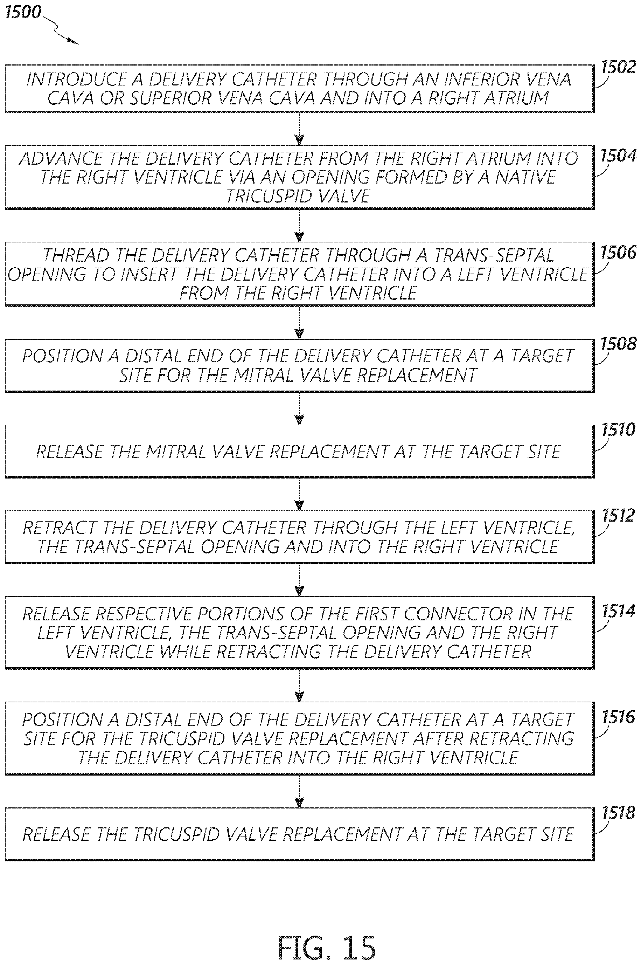

Figure 15 is a flow diagram showing an example of an implantation procedure 1500 for positioning a double heart valve anchoring system as its target site. The double heart valve anchoring system can be carried by a delivery catheter. The double heart valve anchoring system can comprise one or more features described herein (e.g., the double heart valve anchoring systems 100, 110, 120, 130, 140, 150, 190), for example comprising a connector coupling a tricuspid valve replacement and a mitral valve replacement. In some embodiments, the double heart valve anchoring system comprises an anchor such that the connector extends through the anchor. In some embodiments, the double heart valve anchoring system does not include an anchor.

Referring to Figure 15, in block 1502, the delivery catheter can be introduced through an inferior vena cava or a superior vena cava, and into a right atrium. In block 1504, the delivery catheter can be advanced from the right atrium into the right ventricle via an opening formed by a native tricuspid valve. The delivery catheter can be advanced through the right ventricle toward the left ventricle. In block 1506, the delivery catheter can be threaded through a trans-septal opening in an interventricular septum to insert the delivery catheter into the left ventricle from the right ventricle. In some embodiments, the trans-septal opening can be pre-formed, for example being formed prior to insertion of the delivery catheter into the superior vena cava or the inferior vena cava. In some embodiments, the trans-septal opening can be formed by one or more instruments also carried by the delivery catheter. For example, the trans-septal opening can be formed during advancement of the delivery catheter through the septum.

After moving through the trans-septal opening, the delivery catheter can be advanced through the left ventricle toward the native mitral valve. In block 1508, a distal end of the delivery catheter can be positioned at a target site for the mitral valve replacement. In block 1510, the mitral valve replacement can be released at its target site. The mitral valve replacement can be deployed from the distal end of the delivery catheter and positioned at its desired location in the native mitral valve.

After release of the mitral valve replacement, the delivery catheter can be retracted along a path the same as or substantially the same as the path along which it was advanced. In block 1512, the delivery catheter can be retracted through the left ventricle, the trans-septal opening and into the right ventricle. In block 1514, respective portions of the connector can be released in the left ventricle, the trans-septal opening and the right ventricle while retracting the delivery catheter through the left ventricle, the trans-septal opening and into the right ventricle.

As described herein, in some embodiments, the double heart valve anchoring system can comprise an anchor. The anchor can be between the tricuspid valve replacement and the mitral valve replacement and coupled to the connector. In some embodiments, the connector can extend through an opening of the anchor. At least a portion of the anchor and a corresponding portion of the connector can be deployed in the trans-septal opening during retracting of the delivery catheter through the trans-septal opening. The anchor can comprise an occluder configured to facilitate sealing of the trans-septal opening. For example, releasing at least a portion of the anchor and the corresponding portion of the connector in the trans-septal opening can comprise sealing the trans-septal opening. In some embodiments, the trans-septal opening can be sealed once the anchor, comprising a corresponding portion of the connector threaded therethrough, is positioned at its target site.

The delivery catheter can be withdrawn back through the right ventricle toward the right atrium. In block 1516, the distal end of the delivery catheter can be positioned at a target site for the tricuspid valve replacement after retracting the delivery catheter into the right ventricle. In block 1518, the tricuspid valve replacement can be released at its target site. The delivery catheter can be withdrawn through the right atrium and subsequently removed from the heart along the same path as was used in advancing the delivery catheter, such as via the superior vena cava or the inferior vena cava.

As described herein, in some embodiments, a double heart valve anchoring system can comprise a plurality of connectors. For example, the double heart valve anchoring system can comprise one or more additional connectors coupling the tricuspid valve replacement and the mitral valve replacement to one another, such as a second connector. Retracting the delivery catheter through the left ventricle, the trans-septal opening and into the right ventricle can comprise releasing respective portions of both connectors in the left ventricle, the trans-septal opening and the right ventricle. In some embodiments, the double heart valve anchoring system can include an anchor comprising at least a portion configured to be positioned in the trans-septal opening. In some embodiments, both connectors can extend through the anchor. Retracting the delivery catheter through the left ventricle, the trans-septal opening and into the right ventricle can comprise releasing respective portions of both connectors in the left ventricle, the trans-septal opening and the right ventricle, and placing the anchor at its target position in the trans-septal opening.

In some embodiments, a connector can comprise an adjustable length. The length of the connector can be adjusted during the implantation procedure, such as in response to individual anatomy. In some embodiments, one or more imaging techniques can be employed during the procedure in determining an appropriate length of the connector. In some embodiments, the implantation procedure can be performed under guidance of one or more of transesophageal (TEE) echocardiography, transthoracic echocardiography (TTE) and intracardiac echocardiography (ICE). Other known visualization techniques may be employed in addition or in the alternative.

A length of one or more connectors of a double heart valve anchoring system can be adjusted after insertion of the one or more connectors into the patient, with the aid of one or more imaging techniques. The length of the one or more connectors can be selected based on individual anatomy so as to provide desired anchoring to the septum. In some embodiments, the length of a connector can be adjusted such that the connector is taut during ventricular systole.

In some embodiments, the length of the one or more connectors can be adjusted after introducing the delivery catheter into the superior vena cava or inferior vena cava. In some embodiments, the length of the one or more connectors can be adjusted after all replacement valves are positioned at their respective target sites. For example, the length of the one or more connectors can be adjusted after a tricuspid valve replacement is placed at its desired position. In some embodiments, the length of the one or more connectors can be adjusted multiple times. For example, the length of a connector can be adjusted after one or more of positioning a mitral valve replacement, an anchor and a tricuspid valve replacement at their respective target locations. In some embodiments, the length of a connector can be adjusted after one or more of its corresponding portions are released from the delivery catheter. For example, the length of a connector can be adjusted at one or more of: after deployment of the mitral valve replacement and before deployment of the anchor (e.g., after a corresponding portion of the connector has been released in the left ventricle), after deployment of the anchor and before further retraction of the delivery catheter, after deployment of the anchor and before deployment of the tricuspid valve replacement (e.g., after a corresponding portion of the connector has been released in the right ventricle), after deployment of the tricuspid valve replacement. In some embodiments, the length of a connector can be adjusted after each of its corresponding portions are released from the delivery catheter.

A locking mechanism can be deployed, activated and/or triggered to secure a connector at a selected length after the appropriate length has been determined during the implantation procedure. In some embodiments, a locking mechanism can be deployed, activated and/or triggered after each adjustment. As described herein, in some embodiments, the double heart valve anchoring system can comprise multiple locking mechanisms. In some embodiments, a locking mechanism can be deployed, activated and/or triggered after one or more portions of the connector is released and adjusted, including after each portion is released and adjusted.

### Additional Embodiments

Depending on the embodiment, certain acts, events, or functions of any of the processes or algorithms described herein can be performed in a different sequence, may be added, merged, or left out altogether. Thus, in certain embodiments, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain embodiments require at least one of X, at least one of Y and at least one of Z to each be present.

It should be appreciated that in the above description of embodiments, various features are sometimes grouped together in a single embodiment, Figure, or description thereof for the purpose of streamlining the disclosure and aiding in the understanding of one or more of the various inventive aspects.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example embodiments belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A double heart valve anchoring system (100; 120; 130; 140; 150; 160; 170; 180; 190) comprising:
a first anchor (500; 550) comprising at least a portion configured to be secured within a first trans-septal opening in an interventricular septum (10);
a tricuspid valve device coupled to the first anchor (500; 550) via a first connector (400; 420; 430); and
a mitral valve device coupled to the first anchor (500; 550) via the first connector (400; 420; 430).

2. The system of claim 1, wherein the tricuspid valve device comprises a tricuspid valve replacement (200) and the mitral valve device comprises a mitral valve replacement (300).

3. The system of claim 2, wherein the first connector (400; 420; 430) extends between the tricuspid valve replacement (200) and the mitral valve replacement (300), coupling the tricuspid valve replacement (200) and the mitral valve replacement (300) to the first anchor (500; 550), and being configured to extend through the first trans-septal opening.

4. The system of claim 3, further comprising a second connector (400; 420; 430) extending between the tricuspid valve replacement (200) and the mitral valve replacement (300), coupling the tricuspid valve replacement (200) and the mitral valve replacement (300), wherein both the first connector (400; 420; 430) and the second connector (400; 420; 430) couple the tricuspid valve replacement (200) and the mitral valve replacement (300) to the first anchor (500; 550), and are both configured to extend through the first trans-septal opening.

5. The system of claim 4, wherein the system in particular further comprising a second anchor (500; 550) comprising at least a portion configured to be secured within a second trans-septal opening in the interventricular septum (10), and wherein the second connector (400; 420; 430) couples the tricuspid valve replacement (200) and the mitral valve replacement (300) to the second anchor (500; 550).

6. The system of any one of claims 4 to 5, wherein respective ends of at least one of the first connector (400; 420; 430) and the second connector (400; 420; 430) is coupled to a portion of the tricuspid valve replacement (200) configured to be positioned within a right ventricle (4) and to a portion of the mitral valve replacement (300) configured to be positioned within a left ventricle (3),
wherein respective ends of at least one of the first connector (400; 420; 430) and the second connector (400; 420; 430) in particular being coupled to a distal end (202, 204) of the tricuspid valve replacement (200) configured to be positioned within the right ventricle (4) and to a distal end (302, 304) of the mitral valve replacement (300) configured to be positioned within the left ventricle (3).

7. The system of any one of claims 4 to 6, wherein a length of at least one of the first connector (400; 420; 430) and the second connector (400; 420; 430) is adjustable, and
wherein the system in particular further comprising at least one locking mechanism (600) to lock at least one of the first connector (400; 420; 430) and the second connector (400; 420; 430) at a respective selected length.

8. The system of any one of claims 4 to 7, wherein at least one of the first connector (400; 420; 430) and the second connector (400; 420; 430) comprises a rigid connector portion (436), the rigid connector portion (436) being configured to extend through the septum (10) and into at least one of a right ventricle (4) and a left ventricle (3) to provide predetermined angles of septum (10) anchoring for the tricuspid valve replacement (200) and the mitral valve replacement (300) or at least one of the first connector (400; 420; 430) and the second connector (400; 420; 430) is a flexible cord.

9. The system of claim 1, wherein the tricuspid valve device comprises a tricuspid valve replacement (200) and the mitral valve device comprises a mitral valve repair (800) or the tricuspid valve device comprises a tricuspid valve repair (700) and the mitral valve device comprises a mitral valve replacement (300) or the tricuspid valve device comprises a tricuspid valve repair (700) and the mitral valve device comprises a mitral valve repair (800).

10. A double heart valve anchoring system (100; 110; 120; 130; 140; 150; 160; 170; 180; 190) comprising:
a tricuspid valve replacement (200);
a mitral valve replacement (300); and
a first connector (400; 410; 420; 430) coupling the tricuspid valve replacement (200) and the mitral valve replacement (300), and being configured to extend through a first trans-septal opening in an interventricular septum (10) without being coupled to any anchor positioned in and/or through a septum.

11. The system of claim 10, further comprising a second connector (400; 410; 420; 430) coupling the tricuspid valve replacement (200) and the mitral valve replacement (300).

12. The system of claim 11, wherein both the first connector (400; 410; 420; 430) and the second connector (400; 410; 420; 430) are configured to extend through the first trans-septal opening,
wherein the second connector (400; 410; 420; 430) in particular being configured to extend through a second trans-septal opening.

13. The system of any one of claims 11 to 12, wherein respective ends of at least one of the first connector (400; 410; 420; 430) and the second connector (400; 410; 420; 430) are coupled to a portion of the tricuspid valve replacement (200) configured to be positioned within a right ventricle (4) and to a portion of the mitral valve replacement (300) configured to be positioned within a left ventricle (3),
wherein respective ends of at least one of the first connector (400; 410; 420; 430) and the second connector (400; 410; 420; 430) in particular being coupled to a distal end (202, 204) of the tricuspid valve replacement (200) configured to be positioned within the right ventricle (4) and to a distal end (302, 304) of the mitral valve replacement (300) configured to be positioned within the left ventricle (3).

14. The system of any one of claims 11 to 13, wherein a length of at least one of the first connector (400; 410; 420; 430) and the second connector (400; 410; 420; 430) is adjustable, and
wherein the system in particular further comprising at least one locking mechanism (600) to lock at least one of the first connector (400; 410; 420; 430) and the second connector (400; 410; 420; 430) at a respective selected length.

15. The system of any one of claims 11 to 14, wherein at least one of the first connector (400; 410; 420; 430) and the second connector (400; 410; 420; 430) comprises a rigid connector portion (436), the rigid connector portion (436) being configured to extend through the interventricular septum (10) and into at least one of the right ventricle (4) and the left ventricle (3) to provide predetermined angles of septum (10) anchoring for the tricuspid valve replacement (200) and the mitral valve replacement (300) and/or at least one of the first connector (400; 410; 420; 430) and the second connector (400; 410; 420; 430) is a flexible cord.

## Patentansprüche

1. Doppeltes Herzklappenverankerungssystem (100; 120; 130; 140; 150; 160; 170; 180; 190;), umfassend:
einen ersten Anker (500, 550), der mindestens einen Abschnitt umfasst, welcher dazu konfiguriert ist, in einer ersten transseptalen Öffnung in einem interventrikulären Septum (10) befestigt zu werden;
eine Trikuspidalklappenvorrichtung, die mittels eines ersten Verbinders (400; 420; 430) mit dem ersten Anker (500; 550) verbunden ist; und
eine Mitralklappenvorrichtung, die mittels des ersten Verbinders (400; 420; 430) mit dem ersten Anker (500; 550) verbunden ist.

2. System nach Anspruch 1, wobei die Trikuspidalklappenvorrichtung einen Trikuspidalklappenersatz (200) umfasst und die Mitralklappenvorrichtung einen Mitralklappenersatz (300) umfasst.

3. System nach Anspruch 2, wobei der erste Verbinder (400; 420; 430) sich zwischen dem Trikuspidalklappenersatz (200) und dem Mitralklappenersatz (300) erstreckt, den Trikuspidalklappenersatz (200) und den Mitralklappenersatz (300) mit dem ersten Anker (500; 550) verbindet, und dazu konfiguriert ist, sich durch die erste transseptale Öffnung zu erstrecken.

4. System nach Anspruch 3, das ferner einen zweiten Verbinder (400; 420, 430) umfasst, der sich zwischen dem Trikuspidalklappenersatz (200) und dem Mitralklappenersatz (300) erstreckt und der den Trikuspidalklappenersatz (200) mit dem Mitralklappenersatz (300) verbindet, wobei sowohl der erste Verbinder (400; 420; 430) als auch der zweite Verbinder (400; 420; 430) den Trikuspidalklappenersatz (200) und den Mitralklappenersatz (300) mit dem ersten Anker (500; 550) verbinden und beide dazu konfiguriert sind, sich durch die erste transseptale Öffnung zu erstrecken.

5. System nach Anspruch 4, wobei das System insbesondere ferner einen zweiten Anker (500; 550) umfasst, der wenigstens einen Abschnitt umfasst, welcher dazu konfiguriert ist, in einer zweiten transseptalen Öffnung im interventrikulären Septum (10) befestigt zu werden, und wobei der zweite Verbinder (400; 420; 430) den Trikuspidalklappenersatz (200) und den Mitralklappenersatz (300) mit dem zweiten Anker (500; 550) verbindet.

6. System nach einem der Ansprüche 4 bis 5, wobei die entsprechenden Enden von mindestens einem von dem ersten Verbinder (400; 420; 430) und dem zweiten Verbinder (400, 420, 430) mit einem Abschnitt des Trikuspidalklappenersatzes (200) verbunden sind, der dazu konfiguriert ist, im rechten Ventrikel (4) positioniert zu werden, sowie mit einem Abschnitt des Mitralklappenersatzes (300) verbunden sind, der dazu konfiguriert ist, im linken Ventrikel (3) positioniert zu werden,
wobei die entsprechenden Enden von dem mindestens einem von dem ersten Verbinder (400; 420; 430) und dem zweiten Verbinder (400; 420; 430 insbesondere mit einem distalen Ende (202, 204) des Trikuspidalklappenersatzes (200) verbunden sind, das dazu konfiguriert ist, im rechten Ventrikel (4) positioniert zu werden, und mit einem distalen Ende (302, 304) des Mitralklappenersatzes (300) verbunden sind, das dazu konfiguriert ist, im linken Ventrikel (3) positioniert zu werden.

7. System nach einem der Ansprüche 4 bis 6, wobei eine Länge von dem mindestens einem von dem ersten Verbinder (400; 420; 430) und dem zweiten Verbinder (400, 420; 430) einstellbar ist, und
wobei das System insbesondere ferner mindestens einen Verriegelungsmechanismus (600) zur Verriegeln von mindestens dem ersten Verbinder (400; 420; 430) oder dem zweiten Verbinder (400, 420; 430) auf eine entsprechend ausgewählte Länge umfasst.

8. System nach einem der Ansprüche 4 bis 7, wobei mindestens einer von dem ersten Verbinder (400; 420; 430) und dem zweiten Verbinder (400; 420; 430) einen starren Verbinderabschnitt (436) umfasst, wobei der starre Verbinderabschnitt (436) dazu konfiguriert ist, sich durch das Septum (10) in mindestens eines von dem rechten Ventrikel (4) und dem linken Ventrikel (3) zu erstrecken um vorbestimmte Verankerungswinkel am Septum (10) für den Trikuspidalklappenersatz (200) und den Mitralklappenersatz (300) bereitzustellen, oder zumindest einer von dem ersten Verbinder (400; 420; 430) und dem zweiten Verbinder (400, 420; 430) eine flexible Schnur ist.

9. System nach Anspruch 1, wobei die Trikuspidalklappenvorrichtung einen Trikuspidalklappenersatz (200) umfasst und die Mitralklappenvorrichtung eine Mitralklappenausbesserung (800) umfasst, oder die Trikuspidalklappenvorrichtung eine Trikuspidalklappenausbesserung (700) umfasst und die Mitralklappenvorrichtung einen Mitralklappenersatz (300) umfasst, oder die Trikuspidalklappenvorrichtung eine Trikuspidalklappenausbesserung (700) umfasst und die Mitralklappenvorrichtung eine Mitralklappenausbesserung (800) umfasst.

10. Doppeltes Herzklappenverankerungssystem (100; 110; 120; 130; 140, 150; 160; 170; 180; 190), umfassend:
einen Trikuspidalklappenersatz (200);
einen Mitralklappenersatz (300), und
einen ersten Verbinder (400; 410; 420; 430), der den Trikuspidalklappenersatz (200) und den Mitralklappenersatz (300) verbindet und dazu konfiguriert ist, sich durch eine erste transseptale Öffnung in einem interventrikulären Septum (10) zu erstecken ohne mit irgendeinem Anker, der in und/oder durch ein Septum positioniert ist, verbunden zu sein.

11. System nach Anspruch 10, ferner einen zweiten Verbinder (400; 410; 420, 430) umfassend, der mit dem Trikuspidalklappenersatz (200) und dem Mitralklappenersatz (300) verbunden ist.

12. System nach Anspruch 11, wobei sowohl der erste Verbinder (400; 410; 420, 430) als auch der zweite Verbinder (400; 410; 420, 430) dazu konfiguriert sind, sich durch eine erste transseptale Öffnung zu erstrecken,
wobei der zweite Verbinder (400; 410; 420, 430) insbesondere dazu konfiguriert ist, sich durch eine zweite transseptale Öffnung zu erstrecken.

13. System nach einem der Ansprüche 11 bis 12, wobei die entsprechenden Enden von mindestens dem ersten Verbinder (400, 410; 420; 430) oder dem zweiten Verbinder (400; 410, 420; 430) mit einem Abschnitt des Trikuspidalklappenersatzes (200), der dazu konfiguriert ist, in einem rechten Ventrikel (4) positioniert zu werden, verbunden ist und mit einem Abschnitt des Mitralklappenersatzes (300), der dazu konfiguriert ist, in einem linken Ventrikel (3) positioniert zu werden, verbunden ist,
wobei, die entsprechenden Enden von mindestens dem ersten Verbinder (400; 410; 420; 430) oder dem zweiten Verbinder (400; 410, 420; 430) insbesondere mit einem distalen Ende (202, 204) des Trikuspidalklappenersatzes (200), das dazu konfiguriert ist, im rechten Ventrikel (4) positioniert zu werden, verbunden ist und mit einem distalen Ende (302,304) des Mitralklappenersatzes, das dazu konfiguriert ist, im linken Ventrikel (3) positioniert zu werden, verbunden ist.

14. System nach einem der Ansprüche 11 bis 13, wobei eine Länge von mindestens einem von dem ersten Verbinder (400; 410, 420; 430) und dem zweiten Verbinder (400, 410, 420; 430) einstellbar ist, und
wobei das System insbesondere ferner mindestens einen Verriegelungsmechanismus (600) zum Verriegeln von mindestens einem von dem ersten Verbinder (400; 410, 420; 430) und dem zweiten Verbinder (400; 410, 420, 430) auf eine jeweils ausgewählte Länge umfasst.

15. System nach einem der Ansprüche 11 bis 14, wobei mindestens einer von dem ersten Verbinder (400; 410, 420, 430) und dem zweiten Verbinder (400, 410; 420, 430) einen starren Verbinderabschnitt (436) umfasst, wobei der starre Verbinderabschnitt (436) dazu konfiguriert ist, sich durch das interventrikuläre Septum (10) und in mindestens eines von dem rechten Ventrikel (4) und dem linken Ventrikel (3) zu erstrecken, um vorbestimmte Verankerungswinkel am Septum (10) für den Trikuspidalklappenersatz (200) und den Mitralklappenersatz (300) bereitzustellen, und/oder mindestens einer von dem ersten Verbinder (400; 410, 420; 430) und dem zweiten Verbinder (400, 410, 420; 430) eine flexible Schnur ist.

## Revendications

1. Système d'ancrage de valvule cardiaque double (100 ; 120 ; 130 ; 140 ; 150 ; 160 ; 170 ; 180 ; 190) comprenant :
un premier ancrage (500 ; 550) comprenant au moins une partie configurée pour être fixée à l'intérieur d'une première ouverture trans-septale dans un septum interventriculaire (10) ;
un dispositif de valvule tricuspide couplé au premier ancrage (500 ; 550) par l'intermédiaire d'un premier connecteur (400 ; 420 ; 430) ; et
un dispositif de valvule mitrale couplé au premier ancrage (500 ; 550) par l'intermédiaire du premier connecteur (400 ; 420 ; 430).

2. Système selon la revendication 1, dans lequel le dispositif de la valvule tricuspide comprend une valvule tricuspide de remplacement (200) et le dispositif de la valvule mitrale comprend une valvule mitrale de remplacement (300).

3. Système selon la revendication 2, dans lequel le premier connecteur (400 ; 420 ; 430) s'étend entre la valvule tricuspide de remplacement (200) et le la valvule mitrale de remplacement (300), reliant la valvule tricuspide de remplacement (200) et la valvule mitrale de remplacement (300) au premier ancrage (500 ; 550), et étant configuré pour s'étendre à travers la première ouverture trans-septale.

4. Système selon la revendication 3 comprenant en outre un deuxième connecteur (400 ; 420 ; 430) s'étendant entre la valvule tricuspide de remplacement (200) et la valvule mitrale de remplacement (300), reliant la valvule tricuspide de remplacement (200) et la valvule mitrale de remplacement (300), dans lequel à la fois le premier connecteur (400 ; 420 ; 430) et le deuxième connecteur (400 ; 420 ; 430) relient la valvule tricuspide de remplacement (200) et la valvule mitrale de remplacement (300) au premier ancrage (500 ; 550), et sont toutes deux configurées pour s'étendre à travers la première ouverture trans-septale.

5. Système selon la revendication 4, dans lequel le système comprend en particulier un deuxième ancrage (500 ; 550) comprenant au moins une partie configurée pour être fixée dans une deuxième ouverture trans-septale dans le septum interventriculaire (10), et dans lequel le deuxième connecteur (400 ; 420 ; 430) relie le remplacement de la valvule tricuspide (200) et la valvule mitrale de remplacement (300) au deuxième ancrage (500 ; 550).

6. Système selon l'une des revendications 4 à 5, dans lequel les extrémités respectives d'au moins un des premiers connecteurs (400 ; 420 ; 430) et des deuxièmes connecteurs (400 ; 420 ; 430) sont reliées à une partie de la valvule tricuspide de remplacement (200) configurée pour être positionnée dans un ventricule droit (4) et à une partie de la valvule mitrale de remplacement (300) configurée pour être positionnée dans un ventricule gauche (3),
dans lequel les extrémités respectives d'au moins un des premiers connecteurs (400 ; 420 ; 430) et des deuxièmes connecteurs (400 ; 420 ; 430) sont reliées à une extrémité distale (202, 204) de la valvule tricuspide de remplacement (200) configurée pour être positionnée dans un ventricule droit (4) et à une extrémité distale (302, 304) de la valvule mitrale de remplacement (300) configurée pour être positionnée dans le ventricule gauche (3).

7. Système selon l'une des revendications 4 à 6, dans lequel la longueur d'au moins un des premiers connecteurs (400 ; 420 ; 430) et deuxièmes connecteurs (400 ; 420 ; 430) est réglable, et
dans lequel le système comprend en particulier au moins un mécanisme de verrouillage (600) pour verrouiller au moins l'un des premiers connecteurs (400 ; 420 ; 430) et des deuxièmes connecteurs (400 ; 420 ; 430) à une longueur sélectionnée respective.

8. Système selon l'une des revendications 4 à 7, dans lequel au moins l'un des premiers connecteurs (400 ; 420 ; 430) et des deuxièmes connecteurs (400 ; 420 ; 430) comprend une partie de connecteur rigide (436), la partie de connecteur rigide (436) étant configurée pour s'étendre à travers le septum (10) et dans au moins un ventricule droit (4) et un ventricule gauche (3) pour fournir des angles prédéterminés d'ancrage du septum (10) pour la valvule tricuspide de remplacement (200) et la valvule mitrale de remplacement (300) ou au moins un des premiers connecteurs (400 ; 420 ; 430) et des deuxièmes connecteurs (400 ; 420 ; 430) est un cordon flexible.

9. Système selon la revendication 1, dans lequel le dispositif de la valvule tricuspide comprend une valvule tricuspide de remplacement (200) et le dispositif de la valvule mitrale comprend une valvule mitrale de remplacement (800) ou le dispositif de la valvule tricuspide comprend une valvule tricuspide de réparation (700) et le dispositif de la valvule mitrale comprend une valvule mitrale de remplacement (300) ou le dispositif de la valvule tricuspide comprend une valvule tricuspide de réparation (700) et le dispositif de la valvule mitrale comprend une valvule mitrale de réparation (800).

10. Système d'ancrage de valvule cardiaque double (100 ; 110 ; 120 ; 130 ; 140 ; 150 ; 160 ; 170 ; 180 ; 190) comprenant :
une valvule tricuspide de remplacement (200) ;
une valvule mitrale de remplacement (300) ; et
un premier connecteur (400 ; 410 ; 420 ; 430) reliant la valvule tricuspide de remplacement (200) et le la valvule mitrale de remplacement (300), et étant configuré pour s'étendre à travers une première ouverture trans-septale dans un septum interventriculaire (10) sans être couplé à un ancrage positionné dans et/ou à travers un septum.

11. Système selon la revendication 10, dans lequel le deuxième connecteur (400 ; 410 ; 420 ; 430) relie la valvule tricuspide de remplacement (200) et le la valvule mitrale de remplacement (300).

12. Système selon la revendication 11, dans lequel le premier connecteur (400 ; 410 ; 420 ; 430) et le deuxième connecteur (400 ; 410 ; 420 ; 430) sont configurés pour s'étendre à travers la première ouverture trans-septale,
dans lequel le deuxième connecteur (400 ; 410 ; 420 ; 430) est notamment configuré pour s'étendre à travers une deuxième ouverture transseptale.

13. Système selon l'une des revendications 11 à 12, dans lequel les extrémités respectives d'au moins un des premiers connecteurs (400 ; 410 ; 420 ; 430) et des deuxièmes connecteurs (400 ; 410 ; 420 ; 430) sont reliées à une partie de la valvule tricuspide de remplacement (200) configurée pour être positionnée dans un ventricule droit (4) et à une partie de la valvule mitrale de remplacement (300) configurée pour être positionnée dans un ventricule gauche (3),
dans lequel les extrémités respectives d'au moins un des premiers connecteurs (400 ; 410 ; 420 ; 430) et des deuxièmes connecteurs (400 ; 410 ; 420 ; 430) sont reliées à une extrémité distale (202, 204) de la valvule tricuspide de remplacement (200) configurée pour être positionnée dans un ventricule droit (4) et à une extrémité distale (302, 304) de la valvule mitrale de remplacement (300) configurée pour être positionnée dans le ventricule gauche (3).

14. Système selon l'une des revendications 11 à 13, dans lequel la longueur d'au moins un des premiers connecteurs (400 ; 410 ; 420 ; 430) et deuxièmes connecteurs (400 ; 410 ; 420 ; 430) est réglable, et
dans lequel le système comprend en particulier au moins un mécanisme de verrouillage (600) pour verrouiller au moins l'un des premiers connecteurs (400 ; 410 ; 420 ; 430) et des deuxièmes connecteurs (400 ; 410 ; 420 ; 430) à une longueur sélectionnée respective.

15. Système selon l'une des revendications 11 à 14, dans lequel au moins l'un des premiers connecteurs (400 ; 410 ; 420 ; 430) et des deuxièmes connecteurs (400 ; 410 ; 420 ; 430) comprend une partie de connecteur rigide (436), la partie de connecteur rigide (436) étant configurée pour s'étendre à travers le septum interventriculaire (10) et dans au moins le ventricule droit (4) et le ventricule gauche (3) pour fournir des angles prédéterminés d'ancrage du septum (10) pour la valvule tricuspide de remplacement (200) et la valvule mitrale de remplacement (300) et/ou au moins un des premiers connecteurs (400 ; 410 ; 420 ; 430) et des deuxièmes connecteurs (400 ; 410 ; 420 ; 430) est un cordon flexible.
